# EUROPEAN PATENT SPECIFICATION

(11) **EP 1 802 753 B1**
(45) Date of publication and mention of the grant of the patent: **06.10.2010**
(21) Application number: 05789711.8
(22) Date of filing: 18.03.2005
(51) Int. Cl.: C12N 15/10, C12Q 1/68

(54) **METHOD FOR STORING DNA BY USING CHITOSAN, AND PRODUCTS USING THE METHODS**
VERFAHREN ZUR LAGERUNG VON DNA UNTER VERWENDUNG VON CHITOSAN SOWIE PRODUKTE UNTER VERWENDUNG DER VERFAHREN
PROCEDE DE CONSERVATION D'ADN PAR UTILISATION DE CHITOSAN, ET PRODUITS UTILISANT CE PROCEDE

(30) Priority: 07.09.2004 KR 20040071254
(43) Date of publication of application: 04.07.2007
(73) Proprietor: Goodgene Inc., Seoul 133-110 (KR)
(72) Inventor: MOON, Woo-Chul, Busan 600-046 (KR); OH, Myung-Ryurl, Suwon-si, Gyeonggi-do 443-380 (KR); YIM, Su-Bin, Seoul 138-809 (KR); EUM, Tae-Han, Seoul 158-807 (KR); LEE, Mi-Ae, Dong-dae-mun-ku, Seoul (KR); JEON, Bu-Il, Anyang-si, Gyeonggi-do 431-837 (KR)
(74) Representative: Pfenning, Meinig & Partner GbR
(86) International application number: PCT/KR2005/000774
(87) International publication number: WO 2006/028323

(56) References cited:
- EP-A- 1 237 327
- WO-A-03/092739
- JP-A- 2002 238 558
- US-A- 5 985 327
- US-A1- 2001 031 497
- US-A1- 2002 037 094
- MAO H-Q ET AL: "CHITOSAN-DNA NANOPARTICLES AS GENE CARRIERS: SYNTHESIS, CHARACTERIZATION AND TRANSFECTION EFFICIENCY" JOURNAL OF CONTROLLED RELEASE, ELSEVIER, AMSTERDAM, NL, vol. 70, no. 3, 1 January 2001 (2001-01-01), pages 399-421, XP001023009 ISSN: 0168-3659
- KIM T H ET AL: "Efficient gene delivery by urocanic acid-modified chitosan" JOURNAL OF CONTROLLED RELEASE, ELSEVIER, AMSTERDAM, NL, vol. 93, no. 3, 12 December 2003 (2003-12-12), pages 389-402, XP004476727 ISSN: 0168-3659
- KIM T H ET AL: "Galactosylated chitosan/DNA nanoparticles prepared using water-soluble chitosan as a gene carrier" BIOMATERIALS, ELSEVIER SCIENCE PUBLISHERS BV., BARKING, GB, vol. 25, no. 17, 1 August 2004 (2004-08-01), pages 3783-3792, XP004496107 ISSN: 0142-9612
- MADISEN L ET AL: "DNA BANKING THE EFFECTS OF STORAGE OF BLOOD AND ISOLATED DNA ON THE INTEGRITY OF DNA" AMERICAN JOURNAL OF MEDICAL GENETICS, vol. 27, no. 2, 1987, pages 379-390, XP002499895 ISSN: 0148-7299
- ROMOREN KRISTINE ET AL: "Long-term stability of chitosan-based polyplexes" PHARMACEUTICAL RESEARCH (DORDRECHT), vol. 21, no. 12, December 2004 (2004-12), pages 2340-2346, XP002499821 ISSN: 0724-8741
- BORCHARD H: 'Chitosans for gene delivery' ADV DRUG DELIV vol. 52, no. 2, 2001, pages 145 - 150, XP008117497
- KLINE M C ET AL: 'Polymerase chain reaction amplification of DNA from aged blood stains:quantitative evaluation of the "suitability for purpose" of four filter papers as archival media' ANAL CHEM vol. 74, no. 8, 2002, pages 1863 - 1869, XP008116287

## Description

### [Technical Field]

The present invention relates to a method for storing DNA, a method for analyzing the DNA stored by the aforementioned method and products using the methods. More particularly, the invention relates to a method for storing DNA in a stabilized state at room temperature for an extended time, to a method for analyzing the DNA stored by the aforementioned method and to a DNA ID card such as a DNA card made of paper or a DNA card made of plastic, which are produced using the aforementioned storing and analyzing methods.

### [Background Art]

The sum of all genes carried by organisms is referred to as genomes. A human genome is reported to be composed of 3 billion bases and have about 30,000 genes. Recently, the human genome project for decoding base sequences of the full human genome has been completed, thereby allowing epoch-making improvement in diagnosis and treatment of intractable diseases by using genes. So to speak, the time of personalized medicine and predictive medicine has begun.

Life phenomena are determined by three subjects: (1) genetic information of genomic DNA, (2) gene transcription and (3) expressed proteins. In order to understand and analyze life phenomena, and to develop methods for diagnosis and treatment of diseases, it is important to precisely analyze the aforementioned three subjects. The sum of genes in an individual is referred to as genes or genomes, and accordingly the sum of transcribed genes (i.e. mDNA) and the sum of expressed proteins in an individual is referred to as transcriptome and proteome, respectively. Recently, studies on automated analysis of such information in total are progressed actively. For automation of analysis, a microarray or a biochip in particular is helpful, and the representative examples thereof include a DNA chip and a protein chip.

As for a general method for qualitative and quantitative analysis of genetic information of genomic DNA, hybridization analysis, sequencing analysis, DNA microarray or chip, or the like via PCR, PCR-RFLP, cloning and library production, southern blotting or the like (Sambrook, J: & Russell, D.W., Molecular Cloning: A Laboratory Manual, Cold Spring Harbor Press, (2001)) may be mentioned.

When a person's DNA is tested, it is possible to understand the person's health and give a diagnosis of whether the person is contracted to various diseases such as cancer or infection, as well as to predict possibilities of henceforth outbreaks of genetic diseases and transmittance to the person's child. Further, DNA test is the most accurate way to prove one's identity and kinship such as paternity. It is also helpful in proving genealogical lines or family trees. For that reason, DNA test has been used inevitably as a means for the forensic medicine and for discriminating suitability when donating the bone marrow, other internal organs or the like. Further, by analyzing single nucleotide polymorphism (SNP), it may help in predicting possibility of henceforth outbreaks of diseases, reaction to drugs or the like.

As the necessity of storing DNA for future DNA test was recognized, each country has readily established DNA banks, and the number of samples in the DNA bank tends to increase largely in the reason of storing DNA relative to travelers insurances or accident insurances, storing DNA of the U.S. armed forces or the like. However, in storing personal DNA at a national organization or a commercial organization, there may have a problem such as misuse of personal genetic information. Moreover, in order to store DNA safely for an extended time by means of the existing technology, there are need for freezers and specific reagents, thus the cost for storing DNA is large, accordingly.

In fact, when DNA separated and purified from a cell is left to stand at room temperature, there may be a problem of the DNA being rapidly degraded or segmented by deoxyribonuclease (DNAse). Therefore, a method for storing DNA for studies in gene is important. Currently, as for the method of storing or carrying DNA, which is used widely, a method of storing DNA as a liquid state by purifying DNA separated from body fluid such as blood, cells or tissues; a method of storing in a freezer; a method of freeze-dry storing; a method of storing in 2-propyl alcohol; and the like may be mentioned (e.g. Madisen et al., J. Med. Genetics, 27(2), 1987, pp 379-390). However, there are many disadvantages in these methods in the viewpoint of costs and losses. The method of storing the purified DNA as a liquid state is at ease for using the DNA immediately, but there is a problem that the DNA is easily damaged when stored for an extended time. The method of storing in a freezer and the method of freeze-drying has a problem that the DNA may be damaged from repeated melting and freezing in the process of using the sample. Further, the method of storing using a reagent such as alcohol is also troublesome in the process of pre-treatment and post-treatment as well as other methods. Moreover, the most important problem in the above processes is that DNA must be purified first from the body fluid such as blood for storage.

The above-described methods for storing DNA require special expensive equipments such as ultra-low temperature freezer, liquid nitrogen, and the like. Thus, it is disadvantageous in that the methods may impose burden in the economical point of view, the methods require processes of pre-treatment and post-treatment when reusing the stored DNA for analysis, and the method could damage DNA from repeated processes of freezing and defrosting.

In addition to the aforementioned methods, recently, several commercialized products such as a FTA card or a card manufacture by Whatman plc., and the like for the purpose of storing plasmid DNA at room temperature are introduced (e.g. US-A-5985327 published on 16 November 1999). However, this method has limitations to its effectiveness, and it is suggest to store the DNA at -15°C or at -20°C instead of room temperature itself, thus there is no big advantage over the original methods, but rather is disadvantageous in the increase of cost matter.

Therefore, it is an important object in the field of molecular genetics and medicine to develop a possible method for protecting DNA from decomposing by deoxyribonuclease via forming a stable bind with DNA, storing DNA in a stabilized state at room temperature, and further applying directly to the studies and analyses using DNA. Ideal methods of storing should include: (1) a method of storing stably at room temperature without having the DNA degraded; (2) a method should be simple; (3) a method should be cost effective; (4) DNA storing should not only be for the separated and purified DNA itself, but also be for samples containing body fluid such as blood and various DNAs without having damages on the DNA: (5) various DNA samples should be stored in a limited space; (6) an individual should possibly store one's personal DNA; and (7) there should not have any problems in the henceforth analysis on various genes, and these genetic tests should be helpful to actual diagnosis or studies. However, methods or products that satisfy these conditions have not been introduced so far, and reports on the products for storing personal DNA together with the persons genetic information could not be found.

Chitosan is a biologically produced polymer which is excellent in stability, biodegradability and suitability on a living body, and is used widely in the field of medicine. When chitosan is treated with an acid, it forms a water-soluble chitosan salt which becomes charged with a strong positive charge, thus in recent years, the chitosan salt has been noted as a prominent delivering means of drugs, proteins and DNAs. In particular, water-soluble chitosan forms a strong bind with DNA, which is an anionic substance, for the protection of the DNA. Therefore, the water-soluble chitosan may serve as a medium substance for delivering genes.

Chitosan is a copolymer of glucosamine and N-acetylglucosamine and obtained from chitin. Chitin is abundant in crustacean such as crabs or shrimps, and is a substance of natural polymers which is most abundant next to cellulose. Chitosan is a generic term of cationic polymers which is obtained by subjecting chitin to deacetylation with a strong base (Errington N., Harding S.E., Varum K.M., Illum L., Int. J. Biol. Macromol., 15, 1123-1127 (1993)).

Chitosan is not dissolved in neutral or basic pH, while forms chitosan salts or cationic chitosan when reacted with acids such as glutamic acid, hydrochloric acid, lactic acid or the like. The chitosan salt is dissolved in water, and its volubility is proportionate to the degree of deacetylation and pH. In the case of water-soluble chitosan, it becomes positively charged by adding a proton to an amine group. Further, water-soluble chitosan can be made into a freeze-dried state.

Water-soluble chitosan has properties of forming a film or gel with a strong positive charge. In this regard, chitosan is used in purifiers for filthy water, heavy metals and potable water, and antifungal substances. It is also used as raw materials of drinks, health foods and cosmetics. In addition, chitosan is used as an important means in the field of pharmacy. For example, chitosan is used in the wide ranges for delivering of vaccine, delivering of DNA, facilitation of delivering of proteins, peptides and drugs via production by purification and a controlled drug release system, a gel, a film, a wetting agent, a coating agent, a microsphere, a microcapsule, a bioadhesives and a mucous membrane, and the like (Illum L., Pharmaceutical Research, 15, 1326-1331 (1998)).

Chitosan is very safe and show no side effects caused by immunization. When chitosan is absorbed into a living body, it is degraded to N-acetylglucosamine with lysozyme, after which is used in the synthesis of glycoproteins, and then the residual resultant is discharged as a form of carbon dioxide (Chandy T., Sharma C.P., Biomat. Art. Cells Art. Org., 18, 1-24 (1990)).

The most important property of water-soluble chitosan or cationic chitosan according to the invention is that it forms a strong bind with DNA, which is an anionic substance, such that it can be used as a protection and a delivering means of DNA, namely, as a gene delivering substance. In the experiment of using a plasmid DNA, it is reported that chitosan functions as to stably preserve DNA without decomposing by DNA nucleases such as DNase I and DNase II, and that when a complex of chitosan and plasmid DNA is injected into in vitro cultured cell, it triggers expression of a delivering gene (Lee M., Nah J-W., Kwon Y., Koh J.J., Jo K.S., Kim S.W., Pharmaceutical Research, 18(4), 427-531 (2001)). Gene delivering effect is determined by (1) the molecular weight of chitosan and (2) the ratio of chitosan to DNA, in particular, the nitrogen/phosphate ratio of the phosphate molecule of anionic DNA to the nitrogen molecule of cationic chitosan (Borchard G., Advanced Drug Delivering Reviews, 52, 145-150 (2001)).

It is reported that when a plasmid DNA is left to stand at room temperature as it is, the plasmid DNA is decomposed in several hours; however, when a plasmid DNA is kept in a form of a complex with chitosan, it is possible to store the plasmid DNA for 3 months or more (Leong K.W., Mao H.Q., Turong-Lee V.L., Roy K., Walsh S.M., August J.T., J. controlled Rel., 53, 183-193 (1998)). Meanwhile, there has been a report in that when a complex of chitosan and plasmid DNA is stored in a freeze-dried state, the gene delivering effect can be continued until after 4 weeks (Mao H..Q., Turong-Lee V.L., August J.T., Leong K.W., Proc. Intl. Symp. Control. Rel. Bioact. Mater., 24, 671-772 (1997)).

However, the studies on possibility of stably storing large genomic DNA of a mammal with chitosan or the studies on possibility of stably storing DNA with chitosan for an extended time at room temperature has not been reported hitherto. Moreover, products produced to store DNA using chitosan have not been manufactured. Also, possibility of performing an analysis of gene expression using chitosan-bound DNA and subject on storing DNA in a mixed form of chitosan and body fluid, other than DNA itself, such as blood has not been reported. Further, products for storing, carrying and analyzing genomic DNA of a human or an animal using chitosan, other than the products of the invention, have not been released.

### [Disclosure]

### [Technical Problem]

An aspect of the invention is that it provides a method for storing and carrying DNA sample in a stabilized state at room temperature using water-soluble chitosan.

Another aspect of the invention is that it provides a complex of water-soluble chitosan and stabilized DNA which can be used in various methods of DNA test.

A further aspect of the invention according to the method for storing DNA using chitosan of the invention is that it provides a chitosan solution with an appropriate property and concentration that can form a stable bind with DNA in order to possibly carry and store DNA for an extended time at room temperature, easily study and analyze DNA and easily be subject to application.

A still further aspect of the invention is that it provides a method for preparation of a paper type card containing chitosan (a DNA card) that can store combined chitosan and DNA as a liquid state and a number of DNA samples in a minimum space, a method for performing a genetic analysis such as PCR, PCR-RFLP, cloning, sequencing analysis and southern blotting, microarray test and the like, and a method for separating chitosan and DNA from their conbined state. In this case, DNA card can be largely classified into Type 1 and Type 2: Type 1 stores DNA itself on a paper type card containing chitosan, and Type 2 stores bio-sample containing DNA such as blood other than DNA itself on a paper type card comprising chitosan and a cell lysis buffer. In both cases, the object is to store DNA in stabilized state for an extended time at room temperature and to perform various genetic analyses thereafter, if necessary.

Another aspect of the invention is that it provides a DNA identification card (DNA ID card) in which DNA sample of an individual is stored in a form of mixture with chitosan, and genetic information of an individual obtained by performing tests such as a genetic test for personal identification, a HLA genotyping test or the like is saved on a magnetic bar or a chip on the plastic card. The plastic DNA ID card can be used for storing personal information such as various credit cards and cash cards, entrance cards to important organizations, bio-passport, military forces or the like, or sharing genetic information for an organ transplant such as bone-marrow transplantation.

### [Technical Solution]

In the invention, a new method for storing large genomic DNA of animals and plants was studied, and as a result, it was confirmed that when water-soluble chitosan is mixed with DNA, a stable bind is formed so as to protect DNA from decomposing by deoxyribonuclease and can be stored for an extended time in a stabilized state at room temperature, and thus completed the invention.

The conditions to be achieved by the methods and products of the invention are as follows:
1) DNA should be stored in a stabilized state without degrading at room temperature;
2) DNA samples herein should include genomic DNA of humans, animals, plants and bacteria;
3) the method should be simple and should not require special equipments or instruments to be used;
4) the cost should be economical;
5) DNA storing should not only be for the separated and purified DNA itself, but also be for bio-samples containing body fluid such as blood and various DNAs without having damages on the DNA;
6) DNA sample should be stored using a simple medium such as a paper or a card:
7) a number of DNA samples should be stored in a limited space;
8) an individual should possibly store one's personal DNA;
9) with respect to the DNA samples, which was stored and transported by the aforementioned methods and products, various genetic analyses such as PCR, PCR-RFLP, hybridization, sequencing analysis, SNP analysis and the like should be performed accurately and simply even after an extended time, and should be helpful to studies as well as various clinical examinations; and
10) the DNA ID card should function as DNA bank for storing personal DNA, and also store genetic information of the person together therewith.

Hereinafter, the invention will be described in detail in reference to the drawings.

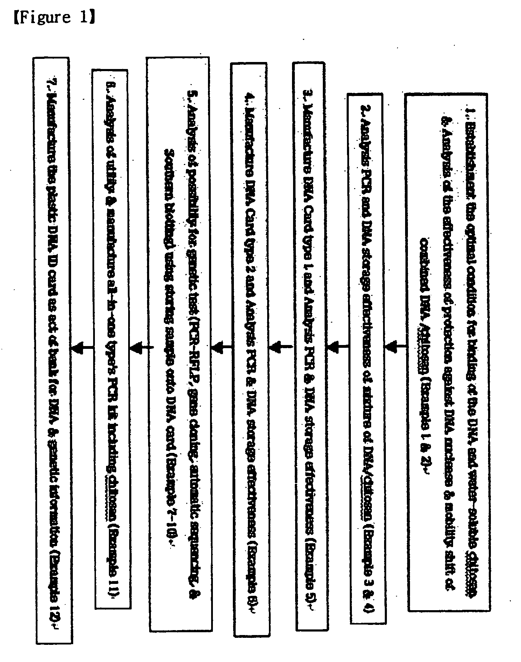

Fig. 1 is a block diagram illustrating the manufacturing and analyzing process of a bound product of DNA and water-soluble chitosan according to the invention. When the process for manufacturing the bound product of DNA and water-soluble chitosan is examined, referring to Fig. 1, first, the optimal condition for binding of the DNA and water-soluble chitosan is established, and the effect of protection against DNA nuclease and mobility shift of the mixture of DNA and chitosan is analyzed (S101). Then, with respect to the mixture of DNA and chitosan as a liquid state, analysis of PCR assay and the effect of storing DNA are conducted (S102). Using the above results, the Type 1 DNA card is manufactured, and analysis of PCR assay and the effect of storing DNA are conducted (S103). In the same manner, Type 2 DNA card is manufactured, and analysis of PCR assay and the effect of storing DNA are conducted (S104). Possibility of genetic analysis based on samples stored for an extended time on the above-manufactured Type 1 and Type 2 DNA card is analyzed (S105). Subsequently, an all-in-one type PCR kit containing chitosan is manufactured, and the availability thereof is analyzed (S106). Then, a plastic DNA ID card is manufactured (S107).

In the invention, in order to form a stable complex with DNA, to protect DNA from decomposing by deoxyribonuclease, and to store DNA in a stabilized state at room temperature, a water-soluble chitosan solution with an appropriate property and concentration, a paper type card or a PCR kit containing such solution and a plastic type DNA ID card and the like are used.

It is preferable that chitosan used in the invention has a molecular weight of 10 to 500 kDa. Chitosan having the molecular weight of less than 10 kDa has problems in forming a stable bind with DNA, and chitosan having the molecular weight exceeding 500 kDa may interfere with analyzing gene expression using DNA. Further, chitosan with the degree of deacetylation of 60% or more is preferred, and when the degree of deacetylation is less than 60%, a problem in water-solubility of chitosan occurs, thus it is difficult to form a stable bind with DNA. The concentration of a prepared aqueous solution of chitosan, in terms of weight to volume ratio (w/v), is 0.02% to 1%, and preferably 0.1%. The mixture ratio at the time of mixing an aqueous solution of chitosan and an aqueous solution of DNA, in terms of the weight ratio of chitosan in the aqueous solution of chitosan to DNA in the aqueous solution of DNA, is 1:0.5 or more, and preferably 1:0.5 to 1:3 (Examples 1 and 2).

DNA/chitosan bound product according to the invention is stored at a temperature from room temperature to -70°C, which is wide in its ranges, and is stored in a dark place without moisture.

Meanwhile, DNA stored in a form of binding with water-soluble chitosan according to the invention is stably preserved even with degradation by deoxyribonuclease is occurred, thus can be used for genetic analysis (see Example 2).

According to the invention, DNAs which can be stored in a mixture with chitosan include genomic DNAs of humans, animals or plants, and bacteria. The size of DNA which can be stored in a mixture with chitosan varies from several tens to several billions of bases.

Meanwhile, a liquid mixture of DNA bound with water-soluble chitosan according to the invention is preserved stably, and the effectiveness of a method for storing DNA of the invention was easily confirmed by performing tests such as polymerase chain reaction (PCR), cloning, blotting, sequencing analysis and the like.

According to the invention, a card for DNA storage (Type 1 DNA card) can be prepared by mixing an appropriate concentration of chitosan and an uric acid (particularly, the volume ratio of the mixture of 0.1% to 1% (w/v) of the water-soluble chitosan solution to the concentration of 0.5 mM to 20 mM of the uric acid is 1:1), and adsorbing the mixture on a paper for blotting, and then drying. Using the aforementioned card, a number of DNA can be stored for extended time and carried in a minimum space at room temperature. As for the paper used in the preparation of the card for storing DNA, various papers for chromatography or papers for blotting that can be used commercially may be used, and the thickness of the paper is preferably about 0.3 mm to 1.2 mm. The storage temperature of the card for storing DNA is suitably room temperature, but lowering the temperature to -20°C and -70°C is allowable, and storing for 6 months or more is possible at room temperature (Example 5).

When performing PCR in the invention, in the case where a mixture of DNA and chitosan is a liquid, the mixture itself is used as a template, and in the case where it is in a card form, a fragment of the card is cut and used as a temperate to perform a general method of PCR amplification, cloning and sequencing analysis. Consequently, it is confirmed that the DNA was preserved stably enough to perform the method of genetic analysis using the stored DNA according to the method of the invention (Example 5).

According to the invention, a card for storing DNA (Type 2 DNA card) can be prepared by adsorbing a mixture of an appropriate chitosan solution with a cell lysis buffer comprising Tris, EDTA, SDS and uric acid (particularly, Tris (8 mM), EDTA (0.5 mM), SDS (0.1% w/v) and uric acid (2 mM)) on a paper for blotting, and then drying. Using the aforementioned card, DNA can be stored for an extended time and carried at room temperature as a bio-sample state containing DNA such as blood (Example 6).

Meanwhile, DNA stored using the DNA card of the invention is preserved stably, and then tests such as PCR, PCR-RFLP, cloning, sequencing analysis and the like can be performed henceforth (Examples 7 to 10).

Further, in order to perform southern blotting, the complex of DNA and chitosan should be separated so that DNA may move on the agarose gel. Thus, DNA is separated by using a sulfate-based cationic salt that has no damage on DNA and has no effect on the results of southern blotting. Examples of the aforementioned sulfate-based cationic salt include sodium dodecyl sulfate (SDS), sodium octyl sulfate (SOS) or cetyltrimethyl-ammonium bromide (CTAB) (Example 9).

As an application of the DNA storage method using chitosan in the invention, a PCR kit was prepared wherein Taq polymerase, a primer, dNTP and buffer and the like were added to one tube with chitosan (all in one tube), and a sample such as DNA or serum was added to the tube to lead to PCR reaction. Characteristically, this PCR kit can be used for both of DNA storage and PCR assay (Example 11).

In addition, the invention provides a DNA ID card in which DNA sample of an individual is stored in a form of mixture with chitosan, and genetic information of an individual obtained by performing a genetic test for personal identification or a HLA genotyping test is saved on a magnetic bar on the plastic card (Examples 12 and 13). The plastic DNA ID card can be used as a personal DNA bank as well as a personal genetic information bank for personal identification, personal organ transplant, personal clinical diagnosis or the like.

### [Description of Drawings]

Fig. 1 a block diagram which shows a process of producing and analyzing a complex product of DNA and water-soluble chitosan according to the present invention.

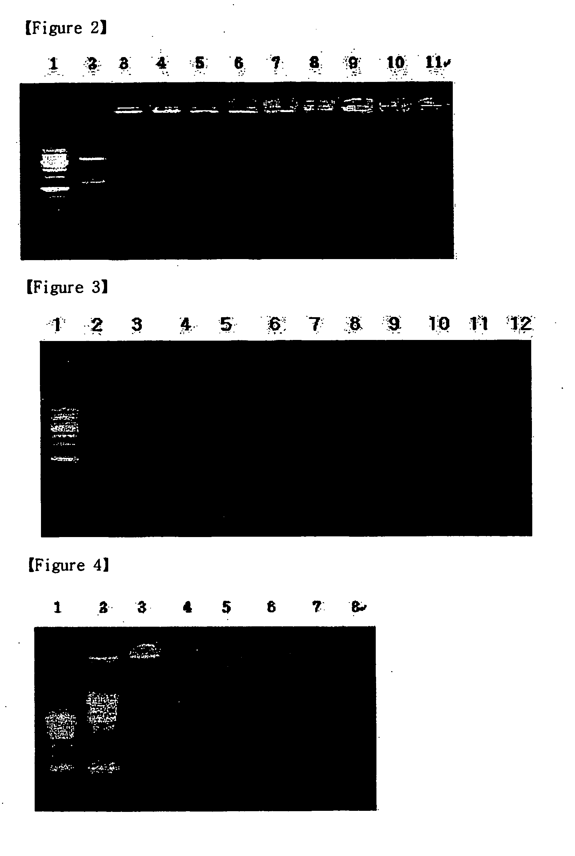

Fig. 2 is a photograph of electrophoresis on a 0.8% agarose gel for a DNA/chitosan complex, which was obtained by mixing genomic DNA isolated from normal adult monocyte at a concentration of 1 µg/µℓ, and a water-soluble chitosan solution at various concentrations with various ratios (Lane 1: size marker (1 kb), Lane 2: only DNA, Lane 3: 0.02% (w/v) of the water-soluble chitosan solution:the aqueous DNA solution = 1:0.2, Lane 4: 0.02% (w/v) of the chitosan solution:the aqueous DNA solution = 1:0.4, Lane 5: 0.02% (w/v) of the chitosan solution:the aqueous DNA solution = 1:0.6, Lane 6: 0.1% (w/v) of the chitosan solution:the aqueous DNA solution = 1:1, Lane 7: 0.1% (w/v) of the chitosan solution:the aqueous DNA solution = 1:2, Lane 8: 0.1% (w/v) of the chitosan solution:the aqueous DNA solution = 1:3, Lane 9: 0.25% (w/v) of the chitosan solution:the aqueous DNA solution = 1:2.5:, Lane 10: 0.25% (w/v) of the chitosan solution:the aqueous DNA solution = 1:5, Lane 11: 0.25% (w/v) of the chitosan solution:the aqueous DNA solution = 1:7.5)
Fig. 3 is a photograph of electrophoresis on a 0.8% agarose gel for a DNA/chitosan complex, which was obtained by mixing a plasmid DNA (pCMV-beta-galactosidase) of 4.0 kb in size at a concentration of 1 µg/µℓ, and a water-soluble chitosan solution at various concentrations with various ratios (Lane 1: size marker (1 kb), Lane 2: only DNA, Lane 3: 0.02% (w/v) of the water-soluble chitosan solution:the aqueous DNA solution = 1:0.2, Lane 4: 0.02% (w/v) of the chitosan solution:the aqueous DNA solution = 1:0.4, Lane 5: 0.02% (w/v) of the chitosan solution:the aqueous DNA solution = 1:0.6, Lane 6: 0.1% (w/v) of the chitosan solution:the aqueous DNA solution = 1:1, Lane 7: 0.1% (w/v) of the chitosan solution:the aqueous DNA solution = 1:2, Lane 8: 0.1% (w/v) of the chitosan solution:the aqueous DNA solution = 1:3, Lane 9: 0.25% (w/v) of the chitosan solution:the aqueous DNA solution = 1:2.5:, Lane 10: 0.25% (w/v) of the chitosan solution:the aqueous DNA solution = 1:5, plane 11: 0.25% (w/v) of the chitosan solution:the aqueous DNA solution = 1:7.5)
Fig. 4 is a photograph which compares and analyzes a degree of DNA cleavage on a 1% agarose gel by treating with deoxyribonuclease (DNAse I) for genomic DNA of human lymphocyte bound to chitosans, and DNA not bound to chitosan (Lane 1: size marker (1 kb ladder), Lane 2: only DNA, Lane 3: chitosan/DNA complex, after 20 minutes, Lane 4: chitosan/DNA complex, after 40 minutes, Lane 5: chitosan/DNA complex, after 60 minutes, Lane 6: DNA not bound to chitosan, after 20 minutes, Lane 7: DNA not bound to chitosan, after 40 minutes, Lane 8: DNA not bound to chitosan, after 60 minutes).

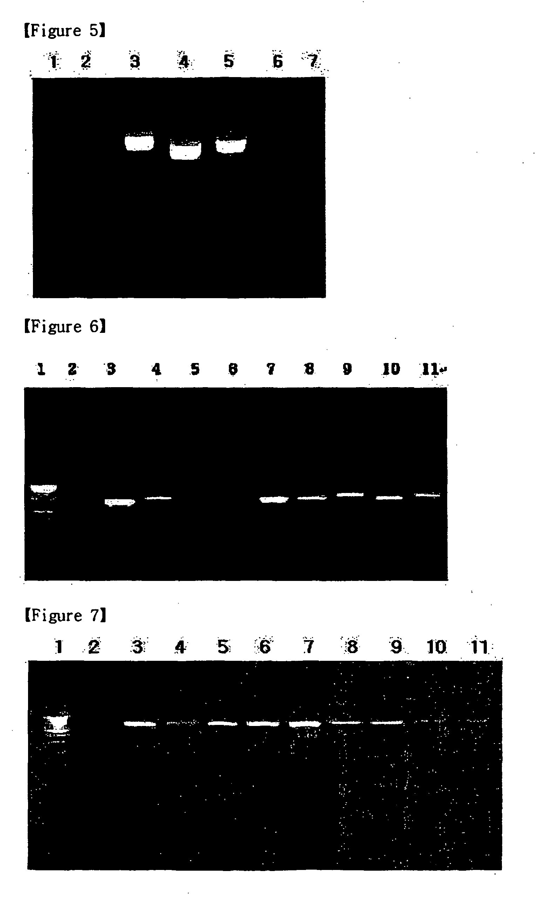

Fig. 5 is a photograph which represents the results of electrophoresis on 0.8% agarose gel of performing PCR for beta-Actin genes with a template of DNA/chitosan complexes, which was obtained by mixing a 0.1% (w/v) chitosan solution and a 1 µg/µℓ concentration of an aqueous genomic DNA solution of human leukocyte at a volume ratio of 1:1, and the genomic DNA of human leukocyte not bound to chitosan, respectively (Lane 1: size marker (100 bp), Lane 2: negative control, Lane 3: positive control (leukocytic genomic DNA was directly analyzed), Lane 4: analysis after dropping the genomic DNA onto the DNA card (Type 1) and then storing it at room temperature for one year, Lane 5: analysis after dropping blood onto the DNA card (Type 2) and then storing at room temperature for one year, Lane 6: analysis after having blood absorbed on the paper for blotting and then storing at room temperature for one year, and Lane 7: analysis after having leukocyte genomic DNA absorbed on the paper for blotting and then storing at room temperature for one year).
Fig. 6 is a photograph which represents the results of electrophoresis on 0.8% agarose gel of performing PCR for beta-Actin genes with a template of DNA/chitosan complexes, which was obtained by mixing a 0.1% (w/v) chitosan solution and a 1 µg/µℓ concentration of an aqueous genomic DNA solution of human leukocyte at a volume ratio of 1:1, and DNA not bound to chitosans, both of which was stored for varied temperatures and times (Lane 1: size marker (100 bp), Lane 2: negative control, Lane 3: positive control (DNA which was isolated and purified from blood, and then immediately PCR was performed therefor), Lane 4: only DNA which was stored at -70°C for three months, Lane 5: only DNA which was stored at room temperature for one week, Lane 6: only DNA which was stored at room temperature for one month, Lane 7: DNA/chitosan complex which was stored at room temperature for three weeks, Lane 8: DNA/chitosan complex which was stored at room temperature for three months, Lane 9: DNA/chitosan complex which was stored at room temperature for one year, Lane 10: Type 1 DNA card with DNA dropped on them, which was stored at room temperature for three weeks, and Lane 11: Type 1 DNA card with DNA dropped on them, which was stored at room temperature for three months).
Fig. 7 is a photograph of agarose gel electrophoresis which represents the results of comparing and analyzing each of the DNA cards by PCR, which were made by adsorbing chitosan on various kinds of blotting papers and drying it to prepare Type 1 DNA card, dropping leukocytic DNA of normal adult thereto with a pipet, and storing them at room temperature for 6 months (Lane 1: size marker (100 bp), Lane 2: negative control, Lane 3: positive control (DNA which was isolated and purified directly from blood), Lane 4: DNA card which was treated with 0.02% chitosan solution on 0.3 mm thick blotting paper, Lane 5: DNA card which was treated with 0.02% chitosan solution on 0.9 mm thick blotting paper, Lane 6: DNA card which was treated with 0.1% chitosan solution on 0.3 mm thick blotting paper, Lane 7: DNA card which was treated with 0.1% chitosan solution on 0.9 mm thick blotting paper, Lane 8: DNA card which was treated with 0.25% chitosan solution on 0.3 mm thick blotting paper, Lane 9: DNA card which was treated with 0.25% chitosan solution on 0.9 mm thick blotting paper, Lane 10: DNA card without treatment on 0.3 mm thick blotting paper, and Lane 11: DNA card without treatment on 0.9 mm thick blotting paper.

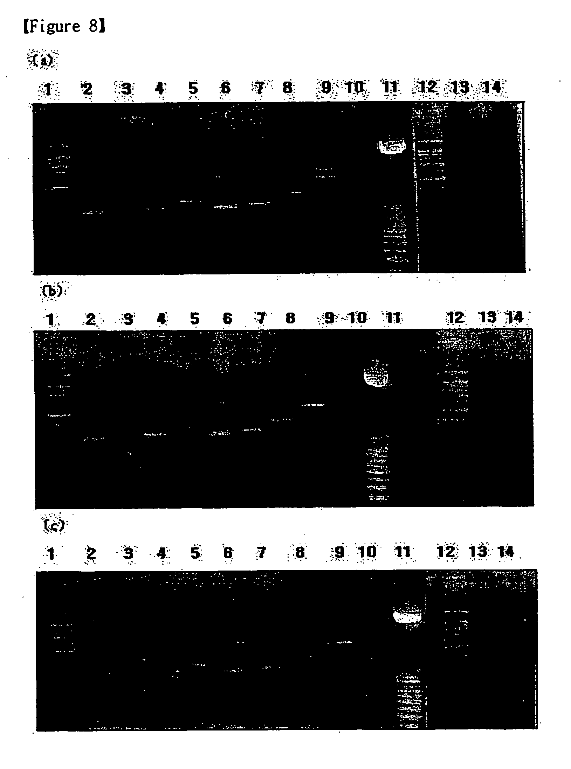

Fig. 8 shows the results of a genotyping test using RFLP assay after PCR for a human genomic DNA sample, which was stored using water-soluble chitosan and the Type 1 DNA card of the present invention ((a): positive control, a DNA sample which is directly isolated and purified from adult leukocyte, (b): a DNA sample which was isolated and purified from adult leukocyte, and mixed with the chitosan solution of the present invention, and stored at room temperature for three months, (c): a DNA sample which was isolated and purified from adult leukocyte, shifted to the Type 1 DNA card of the present invention to be stored, and stored at room temperature for one year) (Lanes 1 and 12: 100 bp size marker, Lane 11: 25 bp size marker, Lanes 2 and 3: assays for Apolipoprotein E (Apo E) genes, Lanes 4 and 5: assays for angiotensinogen (AGT) genes, Lanes 6 and 7: assays for angiotensin converting enzyme (ACE) genes, Lane 8: assay for angiotensin 1 receptor (AT1R) gene, Lanes 9 and 10: assays for endothelial nitric oxide synthase (eNOS) genes, and Lanes 13 and 14: assays for MTHFR genes).

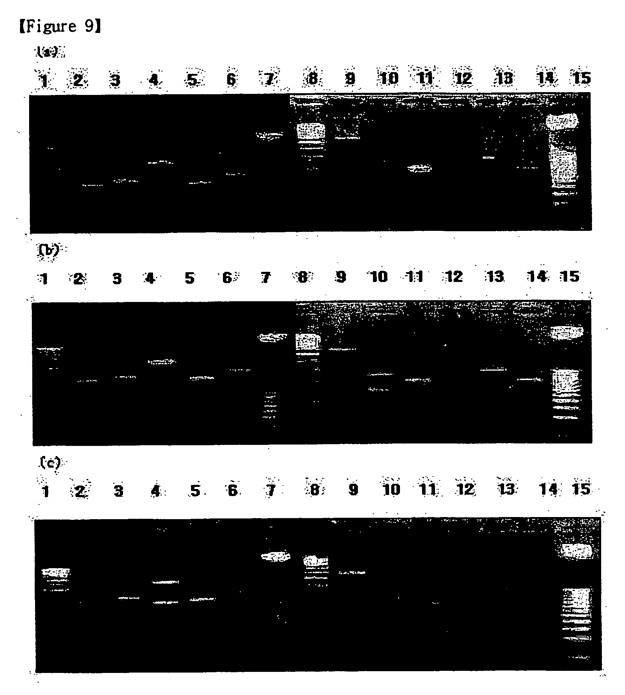

Fig. 9 shows the results of a genotyping test using PCR-RFLP assay for the samples of Type 2 DNA card of the present invention, which were stored while absorbed on it ((a): positive control, a DNA sample which was directly isolated and purified from adult leukocyte, (b): a DNA sample which was made by having adult blood absorbed on the Type 2 DNA card of the present invention and storing it at room temperature for three months, (c) a DNA sample which was made by having adult blood absorbed on the Type 2 DNA card of the present invention and storing it at room temperature) (Lanes 1 and 8: 100 bp size markers, Lanes 7 and 15: 25 bp size markers, Lanes 2 and 3: angiotensinogen genes, Lanes 4 and 5: angiotensin converting enzyme (ACE) genes, Lane 6: angiotensin receptor 1 (AT1R) gene, Lanes 9 and 10: endothelial nitric oxide synthase (eNOS) genes, Lanes 11 and 12: MTHFR genes, and Lanes 13 and 14: Apolipoprotein E (Apo E) genes).

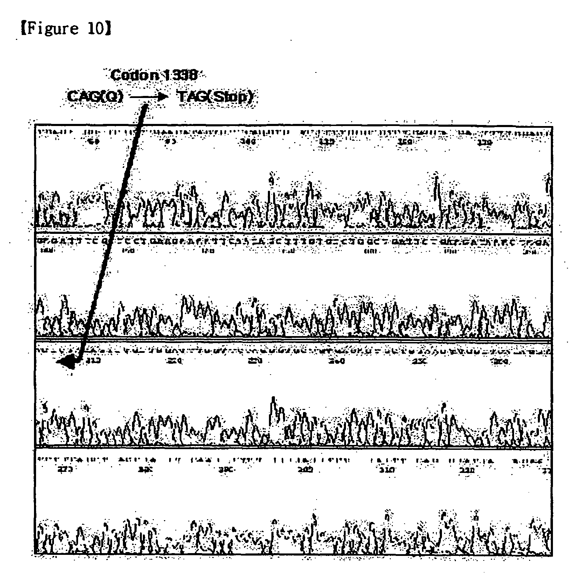

Fig. 10 is a graph which represents the results of searching APC (adenomatous polyposis coli) gene by automatic base sequencing assay, for which PCR is performed, with a template of genomic DNA of adult colic tissue, which was mixed with the water-soluble chitosan solution according to the method of the present invention, and stored at room temperature for one month, and the product thereof was cloned.

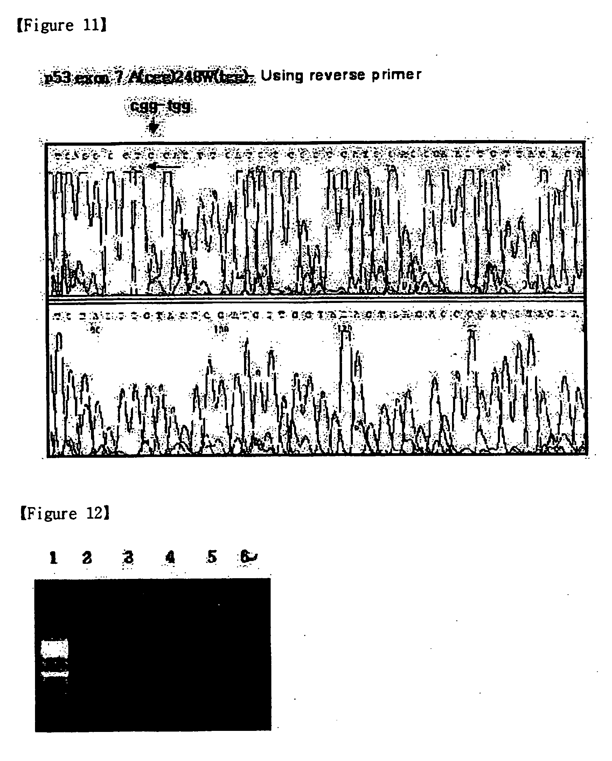

Fig. 11 is a graph which represents the results of searching p53 gene by automatic base sequencing assay, for which PCR is performed, with a template of genomic DNA of lung cancer tissue, which was dropped onto the DNA card of the present invention according to the method and stored at room temperature for three months, and the product thereof was cloned.
Fig. 12 is a photograph of agarose gel electrophoresis which represents the results of isolating chitosan and DNA from a complex of chitosan and DNA using SDS (Lane 1: size marker (100 bp); Lane 2: only DNA, Lane 3: 0.1% (w/v) chitosan solution/aqueous DNA solution (1 µg/µℓ), Lane 4: complex of 0.1% (w/v) chitosan solution/aqueous DNA solution (1 µg /µℓ), which was treated with 0.1% (w/v) SDS, Lane 5: complex of 0.1% (w/v) chitosan solution/aqueous DNA solution (1 µg/µℓ), which was treated with 1% (w/v) SDS, and Lane 6: complex of 0.1% (w/v) chitosan solution/aqueous DNA solution (1 µg/µℓ), which was treated with 5% (w/v) SDS).

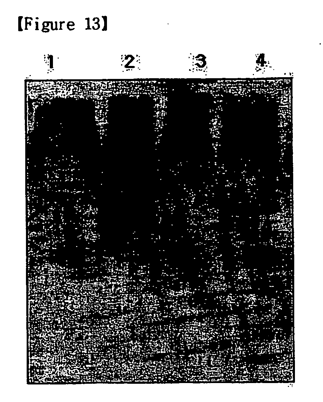

Fig. 13 is a photograph of performing Southern blotting for K-ras gene with a template of a DNA/chitosan complex, which was obtained by mixing an aqueous genomic DNA solution (1 µg/µℓ) of the pancreatic cancer tissue and 0.1% (w/v) chitosan solution at a volume ratio of 1:1 according to the method of the present invention, and storing it at room temperature for three months (Lane 1: DNA of the pancreatic cancer tissue which was stored at room temperature for one month, Lane 2: a complex of pancreatic cancer tissue DNA/chitosan solution which was stored at room temperature for one month, Lane 3: DNA obtained from leukocyte in peripheral blood of normal human, which was stored at -70°C for one month, and Lane 4: DNA obtained from leukocyte in peripheral blood of normal human, which was stored at room temperature for one month).

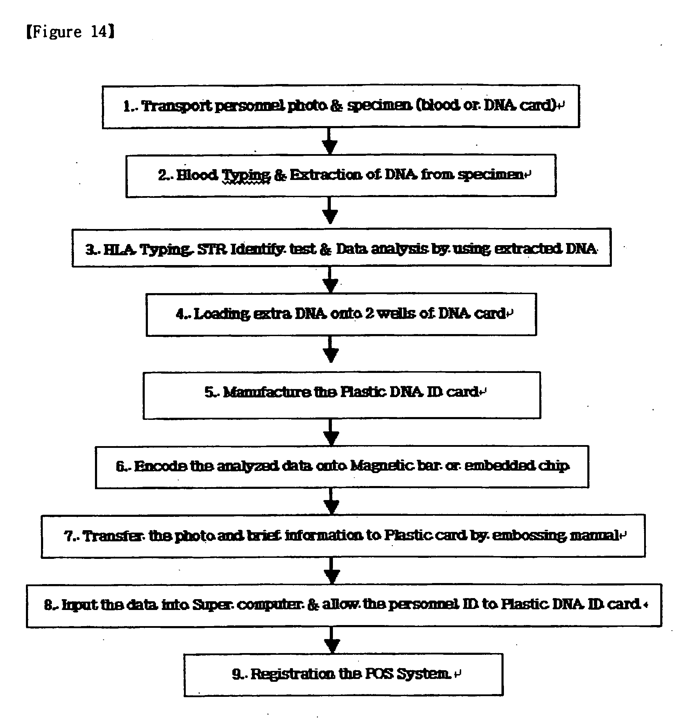

Fig. 14 is a flow chart which shows a process to prepare a plastic DNA ID card which is made using the DNA card of the present invention.

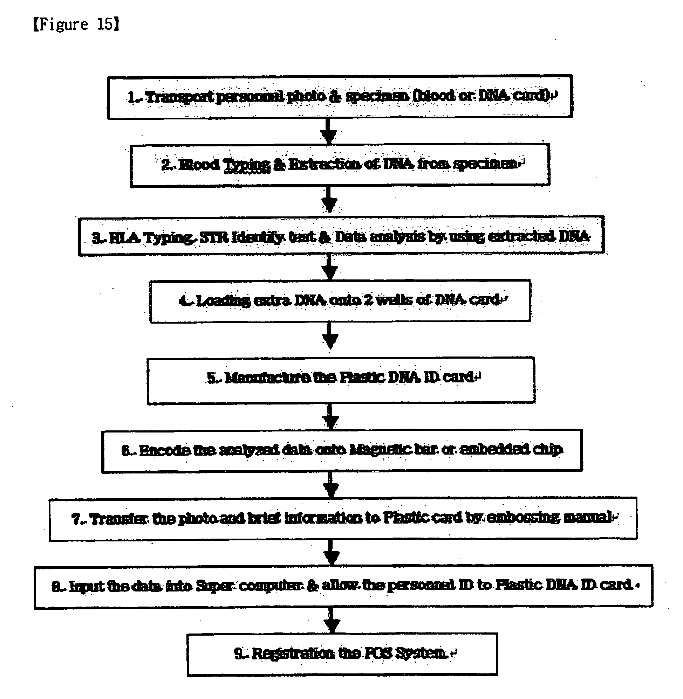

Fig. 15 is a schematic view which shows a process of identifying the personal genotype using 15 combinations of STR test, and preparing a profile of the personal genotype, and encoding the personal genotype data. It is a schematic view of one example of the plastic DNA ID card of the present invention.

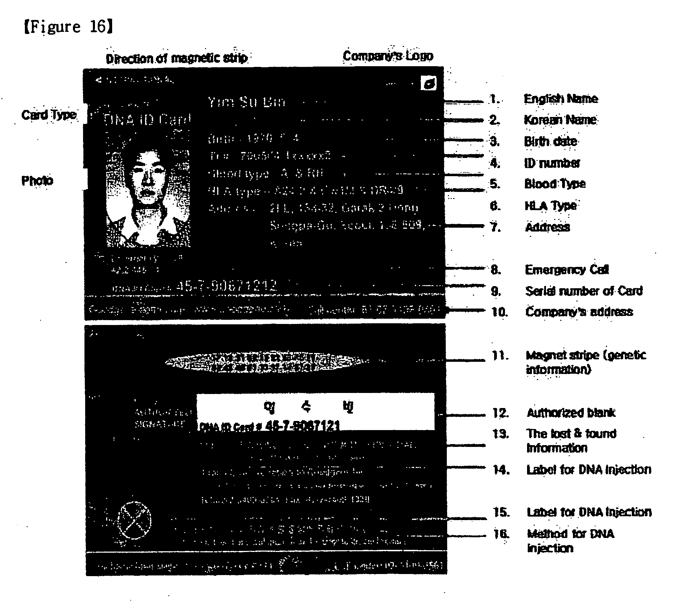

Fig. 16 is a schematic view which shows that Type 1 or Type 2 DNA card is incorporated into the inside of the PVC card in the process of preparing the DNA ID card of the present invention.

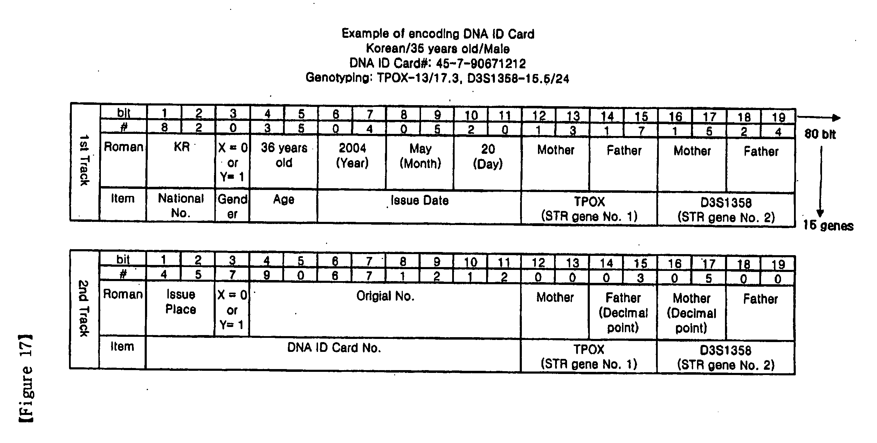

Fig. 17 is a schematic view of one example of the plastic DNA ID card of the present invention.

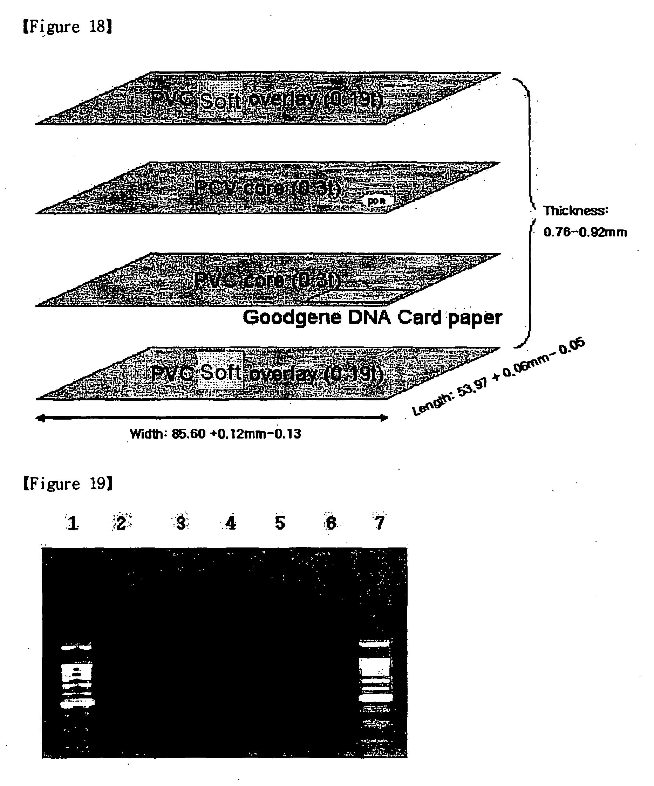

Fig. 18 is a schematic view of one example of encoding information on the DNA ID card of the present invention.
Fig. 19 shows the results of comparing PCR amplifications of eNOS gene, which is one of geriatric disease genes, on a plastic DNA ID card (for blood) which is prepared at high temperature and high pressure, when blood and gDNA were stored on the plastic DNA ID card (Lanes 1 and 7: size markers (100 bp) , Lane 2: DNA ID card on which blood was dropped (for blood), Lane 3: DNA ID card on which gDNA was dropped (for blood), Lane 4: DNA ID card (for blood) on which blood was dropped, and plastic cover, Lane 5: DNA ID card (for blood) on which gDNA was dropped, and a plastic cover, and Lane 6: negative control).

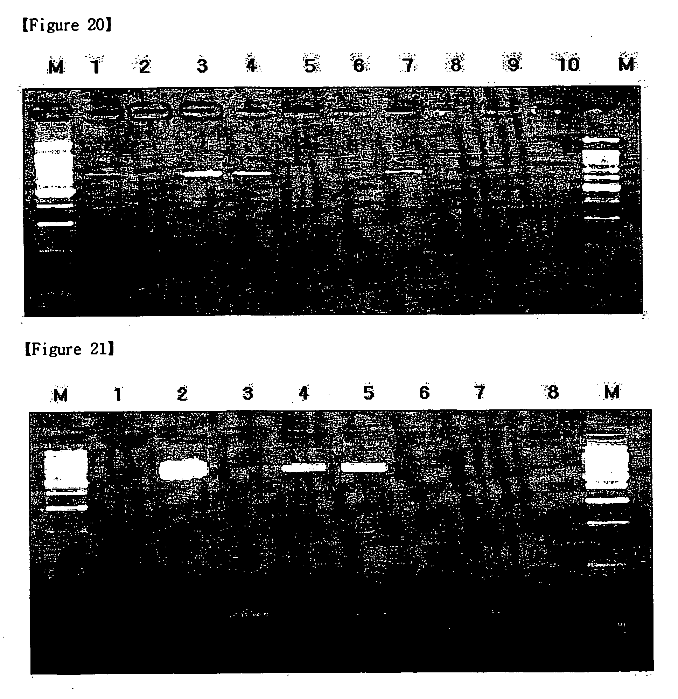

Fig. 20 shows the results of PCR amplifications of eNOS gene, which is one of geriatric disease genes, using plastic DNA ID cards, which were treated with high temperature with no pressure artificially in the laboratory, and those which were prepared practically at high temperature and high pressure, with changing the time to put the sample (Lane 1: DNA ID card for DNA wherein DNA was loaded at room temperature, Lane 2: DNA ID card wherein DNA was loaded after burning at 180°C for 30 minutes, Lane 3: DNA ID card wherein DNA was loaded after burning at 180°C for 1 hour, Lane 4: DNA ID card which was treated at 180°C for 1 hour after loading DNA at room temperature, Lane 5: DNA ID card which was treated at 180°C for 30 minutes after DNA loading, Lane 6: DNA ID card which is treated at 180°C for 1 hour after DNA loading, Lane 7: DNA ID card for blood, which was produced at high pressure (125 bar) and high temperature (180°C), and then loaded with blood, Lane 8: DNA ID card for blood, which was produced at high pressure (125 bar) and high temperature (180°C), and then loaded with DNA, Lane 9: DNA ID card for blood, which was loaded with blood, and then prepared at high pressure (125 bar) and high temperature (180°C), Lane 10: DNA ID card for blood, which was loaded with DNA, and then prepared at high pressure (125 bar) and high temperature (180°C)).
Fig. 21 shows the results of PCR amplification of eNOS gene, which is one of geriatric disease genes using a plastic DNA ID card which was prepared at high temperature and high pressure, specifically, a card fragment for DNA and for blood, which 150 nµg/µℓ gDNA and blood were dropped on, respectively, with changing the dropping amount, and then was stored (Lane 1: negative control, Lane 2: positive control (gDNA of blood), Lane 3: DNA ID card (for DNA) wherein gDNA (5 1) was loaded at room temperature, Lane 4: DNA ID card (for DNA) wherein gDNA (10 1) was loaded, Lane 5: DNA ID card (for DNA) wherein gDNA (50 1) was loaded, Lane 6: DNA ID card (for DNA) wherein blood (10 1) was loaded, Lane 7: DNA ID card (for DNA) wherein blood (50 1) was loaded, Lane 8: DNA ID card (for DNA) wherein blood (100 1) was loaded).

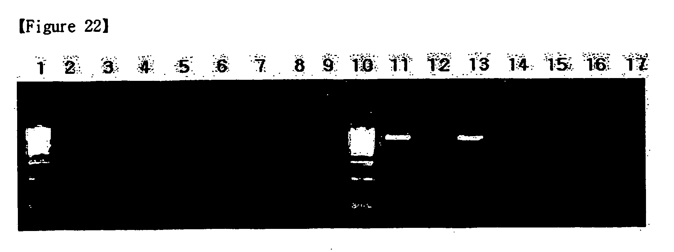

Fig. 22 shows the results of PCR amplification of endothelial nitric oxide synthase (eNOS) gene, which is one of geriatric disease genes, using a sample stored on DNA ID cards which are prepared at high temperature and high pressure, wherein some of the card fragments is treated with a washing buffer as a pretreatment process in PCR amplification, and others not treated (Lanes 1 and 10: 100 bp size markers, Lane 2: negative control, Lane 3: card fragment which was made by treating a collecting card wherein DNA was stored, with a washing buffer at room temperature after four days, Lane 4: card fragment which was made by treating a collecting card wherein. DNA was stored, and BPB (Bromophenolblue) was contained, with a washing buffer at room temperature after four days DNA, Lane 5: card fragment which was made by treating a plastic DNA ID card wherein DNA was stored, with a washing buffer after leaving it at room temperature for four days, Lane 6: card fragment which was made by dropping 0.1% chitosan/DNA complex on 3 mm paper, sterilizing it with autoclave, leaving it at room temperature for four days, and treating it with a washing buffer, Lane 7: card fragment which was made by dropping 0.1% chitosan/DNA complex on a collecting card, leaving it at room temperature for four days, and treating it with a washing buffer, Lane 8: card fragment which was made by dropping 0.1% chitosan/DNA complex on a BPB collecting card, leaving it at room temperature for four days, and treating it with a washing buffer, Lane 9: card fragment which was made by dropping 0.1% chitosan/DNA complex on a plastic DNA ID card, leaving it at room temperature for four days, and treating it with a washing buffer, Lane 11: card fragment which was made by storing a collecting card wherein DNA was stored as a blood state at room temperature for four days, and not treating it with a washing buffer, Lane 12: card fragment which was made by storing a BPB collecting card wherein DNA was stored as blood at room temperature for four days, and not treating it with a washing buffer, Lane 13: card fragment which was made by storing a plastic DNA ID card wherein DNA was stored as blood at room temperature for four days, and not treating it with a washing buffer, Lane 14: card fragment which was made by dropping 0.1% chitosan/DNA complex on 3 mm paper, sterilizing it with autoclave, leaving it at room temperature for four days, and not treating it with a washing buffer, Lane 15: card fragment which was made by dropping 0.1% chitosan/DNA complex on a collecting card, leaving it at room temperature for four days, and not treating it with a washing buffer, Lane 16: card fragment which was made by dropping 0.1% chitosan/DNA complex on a BPB collecting card, leaving it at room temperature for four days, and not treating it with a washing buffer, Lane 17: card fragment which was made by dropping 0.1% chitosan/DNA complex on a plastic DNA ID card, leaving it at room temperature for four days, and not treating it with a washing buffer).

### [Best Mode]

The present invention will be further illustrated with examples below. However, the scope of the present invention is not limited thereto.

### Example 1: Mobility shift assay of a complex of DNA and chitosan through agarose gel electrophoresis

To establish suitable properties, concentration and mixing condition of chitosan for forming a stable complex when mixed with DNA, water-soluble chitosan of various concentrations was mixed with whole DNA isolated from monocyte of normal adult blood in various ratios, and then mobility shift assay of DNA bound to chitosan was carried out through agarose gel electrophoresis. Furthermore, mobility shift assay of DNA bound to chitosan was carried out in the same manner as for genomic DNA of mouse hepatic tissue and plasmid DNA (pCMV-beta-galactosidase) of 4.0 kb size. The method for experiment and the results are as follows.

### A. DNA isolation and preparation

Whole DNA of the cell from normal adult leukocyte was isolated, the experiment was performed using tertiary distilled water according to a known method (Sambrook J & Russell DW, Molecular cloning: a laboratory manual, Cold Spring Harbor Press. 2001:7.1-7.88).

The peripheral venous blood was collected in a tube containing sodium citrate (vaccutainer CTAD tube, catalogue No #367946, Becton & Dickinson, USA), and the plasma and buffy coat layer of the monocyte were separated through centrifuge. The monocyte DNA, most of which is lymphocyte, was isolated using a QIAamp DNA blood minikit (Qiagen, Germany) in the following method.

20 µℓ of protein kinase K (QIAGEN protease) was put into 1.5 ml-microcentrifuge tube, 200 µℓ of the buffy coat layer and 200 µℓ of buffer AL were sequentially added thereto, and mixed by vortex for 15 seconds. It was left to stand at 56°C for 10 minutes and centrifuged to collect the solution stuck to the tube cap. 200 µℓ of ethanol was added thereto, mixed by vortex for 15 seconds, and all of the solution was added to a QIAamp spin column, and was centrifuged at 8,000 rpm for one minute. Then, a 2 ml-collecting tube was inserted into the column, 500 µℓ of a buffer AW1 was added thereto, and centrifuged again at 8,000 rpm for one minute. Thus-obtained filtrate was discarded, and a new tube was provided, and further centrifuged at a maximum speed for one minute. Again, a new 1.5-ml microcentrifuge tube was inserted, 200 µℓ of distilled water or a buffer AE was added thereto, left to stand at room temperature for one minute, and then further centrifuged at 8,000 rpm for one minute to elute DNA. Thus-isolated DNA was measured on the concentration using a spectrophotometer, and the A260/A280 ratios were compared in order to find the purity of the isolated DNA. At this time, the DNA purity should be such that A260/280 is 1.6 to 1.8 upon spectrophotometer measurement. From the above method, 6 µg on average of pure DNA can be obtained from 200 µℓ of human blood.

### B. Preparation of chitosan

If a water-soluble chitosan is a product of which the deacetylation degree is 60% or more and the molecular weight is 10 kDa to 500 kDa, it can be used for the applications of the present invention, specifically for forming a stable complex with DNA. Many water-soluble chitosan products corresponding to these are commercially available. Among them, in the present invention, a water-soluble chitosan product of which the average deacetylation rate is 90.1% and the average molecular weight is about 300kDa, was purchased from Jakwang Co. Ltd. (Ansungsi, Republic of Korea), and dissolved in sterilized tertiary distilled water. Besides this chitosan, other water-soluble chitosan having similar properties may be also used.

### C. Method for preparation of a complex of chitosan and DNA

The water-soluble chitosan was dissolved in sterilized tertiary distilled water, and prepared at various concentrations of from 0.02% to 0.05%, 0.1%, 0.25%, 0.5% and 1% of weight versus volume ratio (w/v) for test. Here, DNA was diluted with sterilized tertiary distilled water to the concentrations of 500 nµg/µℓ and 1 µg/µℓ, and then used.

In a 1.5-ml centrifuge tube, a DNA solution and the chitosan solution of various concentrations as described above were mixed at various volume ratios, and left to stand at room temperature for 15 minutes to induce formation of a complex.

### D. Mobility shift assay of a complex of DNA and chitosan through agarose gel electrophoresis

The DNA/chitosan complex obtained by mixing a 1 µg/µℓ concentration of genomic DNA isolated from normal adult monocyte and plasmid DNA, and the water-soluble chitosan solutions of various concentrations at various ratios were loaded on 0.8% agarose gel according to the above method, and subjected to electrophoresis at 100 V. The results were shown in Figs. 2 and 3, respectively. The results showed that when only DNA which was not mixed with chitosan, was electrophoresed, DNA shifted normally and rDNA bands were observed at 18s and 28s, whereas an aqueous DNA solution (1 µg/ µℓ) was mixed with a 0.02%, 0.1% or 0.25% chitosan solution, respectively at each volume ratio of 1:0.2, 1:1 or 1:2.5 or more (at this time, weight ratio of chitosan and DNA in the mixture is 1:1 or more), DNA was bound completely to chitosan such that it never shifted on the agarose gel. Therefore, it was made as a standard in the following experiment that the composition of a complex of chitosan and DNA was such that 0.1% chitosan solution was mixed with the DNA solution of a 1 µg/µℓ concentration at a volume ratio of 1:1 (at this time, the weight ratio of chitosan and DNA in the mixture is 1:1). However, this is only one example of the composition, and the suitable composition may be somewhat varied depending on the properties of the chitosan product used.

### Example 2 Deoxyribonuclease protection assay of chitosan

To ascertain if the water-soluble chitosan can protect DNA from deoxyribonuclease (DNAse), and establish the suitable conditions, DNA which was treated with a chitosan solution, and which was DNA not treated, were treated with deoxyribonuclease, respectively, and the effect was observed through agarose gel electrophoresis.

The method and .results were as follows. The same experiment was performed repeatedly with genomic DNA of human lymphocyte and genomic DNA of mouse hepatic tissue.

Eight tubes were divided into two groups. In one group, chitosan (20 g) dissolved in distilled water and DNA (10 g) were mixed in a microcentrifuge tube (1.5 ml) to produce a complex. In the other group, only DNA (10 g) was added to the tube without chitosan. To each of the tubes of the first group and the second group, 5 µℓ of deoxyribonuclease (DNAse I), which was dissolved at a concentration of 1 unit/1, was added, and then distilled water was added to 100 µℓ, pf a final volume. Then, they were held for reaction in 37°C incubator for 0 minute, 20 minutes, 40 minutes and 60 minutes, respectively. Immediately after the reaction finished, 25 µℓ of the stop solution was added and well mixed to inactivate DNAse I. To each of the four tubes wherein the reaction was inactivated, 110 µℓ of TE buffer (10 mM Tris, 0.1 mM EDTA) was added and mixed, and then they were held for reaction at 60°C overnight. Then, 150 µℓ of phenol/chloroform was added and mixed by vortex for one minute, and centrifuged at room temperature for 5 to 8 minutes. Then, a supernatant was taken and put into a new microcentrifuge tube, and extracted. 300 µℓ of anhydrous EtOH and 15 µℓ of 3M NH40AC were added thereto, and left to stand previously at -70°C in a deep freezer for 20 to 30 minutes. Then, the resultant was centrifuged at 4°C in 12,000 rpm for 20 minutes, and the liquid was discarded by suction. The settled pellet was washed again with 70% ethanol, and dried, and dissolved in 20 µℓ of distilled water, which was free from DNAse. 10 (about 1 µg DNA) of the resultant was taken from thus-obtained total eight tubes, respectively, and subjected to electrophoresis on 1% agarose gel. The results were shown in Fig. 4.

From Fig. 4, it was found that DNA which was not treated with chitosan, was almost cleaved 40 minutes after treatment with DNAse I, whereas DNA which was treated with chitosan, was not cleaved 40 minutes after treatment with DNAse, most of which remained in the wells. These results show that if chitosan and DNA are mixed even at a 1:0.5 weight ratio according to the method of the present invention, it also suppresses effectively the DNA cleavage by a deoxyribonuclease, and the method according to the present invention is effective for DNA storage.

### Example 3: Investigation of gene amplification possibility for a complex of DNA and chitosan

Using the liquid complex of the water-soluble chitosan and DNA prepared according to Examples 1 and 2, PCR was performed to ascertain if the target gene is appropriately amplified, and thereby to ascertain if the DNA storage method of the present invention has no harmful effect on the gene assay, and to establish suitable conditions for using DNA stored by the DNA storage method according to the present invention in PCR. For human leukocytic genomic DNA, PCR was performed with the target of each ? actin gene. The method and results were as follows.

PCR reaction was performed using 100 ng of the subject DNA as a template by adding 10 pmol of the forward primer and the reverse primer of the target gene, respectively, and further adding 2.5 mM deoxynucleotide (dNTPs), 1 unit Taq polymerase and 10 x polymerase buffer (500 mM KCl, 100 mM Tris-Cl, 15 mM MgCl₂, 0.1% gelatin). PCR was performed using GeneAmp 2700 thermal cycler (Perkin-Elmer Biosystems, Inc., USA), firstly under the condition of first 95°C for 5 minutes, then forty times under the condition of 95°C for 30 seconds, 55°C for 30 seconds and 72°C for 30 seconds, and finally at 72°C for 7 minutes, to give PCR products.

With each template of genomic DNA samples of human and mouse prepared by mixing 0.1% chitosan and DNA (1 µg/µℓ) at a volume ratio of 1:1, and DNA which was not mixed with chitosan, PCR was performed for beta-Actin gene. The results of electrophoresis on 1.5% agarose gel were shown in Fig. 5. Both of them which were mixed with chitosan showed a strong DNA band of beta-Actin gene of 661 bp size in the same degree. This shows that the chitosan-mixed medium of the present invention has no harmful effect on PCR, and can be used usefully for searching gene expression.

### Example 4: PCR amplification experiment of a complex of DNA and chitosan, which was stored at room temperature for a long time

From the above results, it was found that the water-soluble chitosan is nearly completely bound to DNA, and preserves DNA stably from the attack of deoxyribonuclease. In the present example, it was ascertained if when the complex of the water-soluble chitosan and DNA was stored in the liquid form at room temperature for a long time, the stored DNA is kept with no cleavage to make it possible to perform gene amplification and assay. It was performed by PCR assay as follows.

1 µℓ of 0.1% the water-soluble chitosan solution (w/v) and 1 µℓ of genomic DNA of monocyte of normal adult blood (500 nµg/µℓ) were mixed to form a complex, and 8 µℓ of distilled water was further added to adjust the final volume to 10 µℓ. Thus, 24 samples were prepared in the centrifuge tubes and stored at room temperature. 1 µℓ of the same leukocyte DNA, which is not treated with chitosan, was adjusted to a final volume 10 µℓ, to prepare 9 samples. Three of them were stored at -70°C and the remaining six samples were stored at room temperature. For the DNAs which were mixed with the water-soluble chitosan and stored in the tube at room temperature, PCR reaction was performed according to the method of Example 3 for three DNA samples, respectively at the time point of one week, two weeks, three weeks, four weeks, three months, six months, nine months and one year, respectively to ascertain if the target gene is appropriately amplified. For the three samples which were stored at -70°C among the DNA samples which were not mixed with chitosan, PCR was performed after three months, and for each three DNA samples which were stored at room temperature, PCR was performed after three months and one year, respectively. Fig. 6 is a photograph which represents the results of electrophoresis on 0.8% agarose gel for beta-Actin gene after performing PCR.

From Fig. 6, it was found that when DNA was stored in a microcentrifuge tube at room temperature for three months and for one year as not bound to chitosan (naked), beta-Actin, which is a housekeeping gene, was never expressed. On the contrary, when the DNA sample was stored as bound to chitosan in a tube as a liquid at room temperature for one week to one year, beta-Actin gene was clearly detected. It was found that for the DNA sample which was stored at -70°C without bound to chitosan according to the conventional method, the amount of amplified beta-Actin is similar to that of the DNA sample which was stored with bound to chitosan at room temperature according to the method of the present invention.

Such results showed that the method of storing DNA in the liquid as bound to the water-soluble chitosan according to the method of the present invention, can keep DNA stably for at least one year, that the stored DNA has no problem in being used in PCR assay, and that the method can store DNA stably similarly to the conventional storage method of the deep freezer. The storage temperature in the present example varies from room temperature to -70°C. It is preferably stored in non-humid dark place.

### Example 5: Preparation of DNA card (Type 1) where chitosan is adsorbed on paper, and investigation of gene amplification possibility therefor

To store and carry the multiple sample DNAs at room temperature in a minimum space, and help with automatic assay, a card wherein chitosan is absorbed on a suitable paper has been developed, which was designated as a DNA card. The DNA card is in various kinds, and among them, a paper card of which the use is storing DNA itself, was designated as Type 1 DNA card.

To select the most effective concentration of the water-soluble chitosan solution and appropriate thickness of the paper, the following experiment was performed.

Various kinds of papers for blotting were submerged in 0.02%, 0.1% or 0.25% (w/v) concentration of the water-soluble chitosan solution during a sufficient time period, and dried to be used in producing DNA cards. At this time; the paper is selected from papers for blotting which are commercially available from various companies such as Whatman plc. (UK) or Schleicher & Schuell GmbH (Germany), and has a suitable thickness. In the present invention, papers for blotting of 0.3 mm, 0.9 mm and 1.2 mm in thickness were purchased from Whatman plc. and used. The kind and size of the DNA card for DNA storage with chitosan can be prepared variously depending on use. The blotting paper on which chitosan is adsorbed, was cut to the same size of 24-well, 96-well and 384-well plate (multiwell-plate) (8.1 cm 12.3 cm), which are generally well used, and the well and partition were printed and marked on the paper like the plate. On each of the wells, a DNA sample was dropped with a pipet, and stored for one week to one year or more at room temperature. Thus, one DNA card can store 24 to 96, to 384 pieces of DNA samples.

A method of producing the water-soluble chitosan-submerged DNA card is as follows.

A 0.3 mm thick paper for blotting from Whatman plc. was sterilized and dried in an autoclave at high temperature. Then, 0.1% (w/v) concentration of water-soluble chitosan solution and 2 mM uric acid solution, which were previously produced, were mixed at a volume ratio of 1:1, and the paper was submerged during a sufficient time period, and then dried to produce Type 1 DNA card.

On the Type 1 DNA card prepared as above, human genomic DNA in which human genome DNA was spotted and stored for a long time, was dropped, and stored for a certain period. From the stored DNA card, a card fragment of about 1 mm to 2 mm in diameter was taken using a punch, a forceps or a pincette, and added to a tube for performing PCR. With a template of this card fragment, PCR for beta-Actin gene was performed according to the method of Example 3. The method of this PCR reaction was as follow.
1. From a chitosan-submerged paper card wherein DNA is stored, a fragment of about 1.2 mm in diameter was taken using a punch, etc. and added to a tube for PCR.
2. 200 µℓ of a TE buffer (10 mM Tris-Cl, 0.1 mM EDTA, pH = 8.0) was added, mixed, and then left at room temperature for 5 minutes. Thereafter, the TE buffer was completely removed using a pipet.
3. The second process was repeated twice.
4. It was dried at room temperature for 1 hour or at 56°C for 10 minutes.
5. To the card fragment which was treated by the above processes, a PCR sample of a suitable composition was added and beta-Actin gene was PCR amplified according to the method of Example 3.

Fig. 7 shows the results of a photograph of agarose gel electrophoresis experiment, which represents results of comparison and analysis by PCR. It was found that for all of DNA cards, which were made by being submerged in a 0.1% chitosan solution, the target gene was strongly expressed, and that the thicker the paper was such as 0.9 mm or 1.2 mm, the more PCR products were produced. In addition, when stored as adsorbed on the DNA card of the present invention, beta-Actin gene was strongly amplified in PCR even after a long time such as three months to one year at room temperature similar to the case of storage at -70°C.

Such results show that when stored in the DNA card according to the invention, multiple various DNA samples can be stably preserved even at room temperature for a long time, and can be assayed after a long time. Therefore, it was found that the DNA card of the invention is useful for storing and carrying multiple various DNA samples simply at room temperature for a long time, and further also useful for various gene assays such as PCR or base sequencing as shown in the present example and the following examples.

### Example 6: Preparation of a DNA card (Type 2) wherein chitosan and cell lysis buffer are adsorbed on the paper and investigation of gene amplification possibility therefor

This Type 2 DNA card was produced by adsorbing chitosan and a cell lysis buffer on various kinds of papers for blotting and drying it. This Type 2 DNA card was designed to be used to store samples as cell-containing body liquid such as blood, and then amplify them by PCR for gene assay. Onto this Type 2 DNA card, human blood was dropped, and after a certain period, the performance was compared and analyzed by PCR.

The paper card for blotting prepared as in Example 5 was submerged in the water-soluble chitosan solution and cell lysis buffer during a sufficient time period and dried to produce a DNA card. At this time, the papers for blotting of 0.3 mm, 0.9 mm and 1.2 mm in thickness were purchased from Whatman plc. and used. A method of producing Type 2 DNA card for storage as blood is as follows.

0.3 mm thick paper for blotting from Whatman plc. was sterilized at high temperature and dried. Then, 0.1% (w/v) concentration of the water-soluble chitosan solution and cell lysis buffer (0.5 mM EDTA, 8 mM Tris-Cl, 2 mM uric acid, 1% (w/v) SDS), which were previously produced, were mixed at a volume ratio of 1:1, and the paper was submerged during a sufficient time period, and then dried to produce Type 2 DNA card.

From the above DNA card, a card fragment of about 1 mm to 2 mm in diameter was taken, and added to a tube for performing PCR as in Example 5. With a template of this card fragment, PCR for beta-Actin gene was performed. The method of this PCR reaction was as follows.
1. From a chitosan-submerged paper card wherein DNA is stored, a fragment of about 1.2 mm in diameter was taken using a punch, etc. and was added to a tube for PCR.
2. 200 µℓ of a washing solution (GG purification reagent; 0.5 mM EDTA, 8 mM Tris-Cl, 2 mM Uric acid, 1% (w/v) SDS) was added, mixed and left at room temperature for 5 minutes. Then, the washing solution was completely removed using a pipet.
3. The second process was repeated three times.
4. Then, 200 µℓ of a TE buffer (10 mM Tris-Cl, 0.1 mM EDTA, pH = 8.0) was added, mixed and left at room temperature for 5 minutes. Then, the TE buffer was completely removed using a pipet.
5. The fourth process was repeated twice or three times.
6. It was dried at room temperature for 1 hour or at 56°C for 10 minutes.
7. To the card fragment which was treated by the above processes, a PCR sample of a suitable composition was added, and beta-Actin gene was PCR amplified in the same manner as in Example 2.

As results of the experiment, it was found that for all of Type 2 DNA cards, the target gene was strongly expressed, and that the thicker the paper was such as 0.9 mm or 1.2 mm, the more PCR products were produced. In addition, when stored as adsorbed on the DNA card of the invention, beta-Actin gene was strongly amplified in PCR even after a long time such as three months to one year at room temperature. Such results show that DNA sample can be stably preserved at room temperature for a long time as blood with no need to isolate and purify DNA when stored in the DNA card according to the invention, and can be assayed after a long time. Therefore, it was found that the DNA card of the invention is useful for storing and carrying simply DNA samples as blood at room temperature for a long time, and further also useful for various gene assays such as PCR or the like.

### Example 7: Cloning of target gene for a complex of DNA and chitosan stored for a long time and investigation of base sequencing possibility

PCR was performed for a chitosan/DNA complex which was stored as a liquid at room temperature for a long time according to Example 4, and the obtained product was cloned. Then, it was ascertained by automatic base sequencing if the DNA stored according to the method of the invention can be stably kept and used after the storage.

With a template of a genomic DNA sample of an adult colic tissue, which was stored as mixed with 0.1% the water-soluble chitosan according to the method of the invention at room temperature for three months, PCR was performed for adenomatous polyposis coli (APC) gene. The product was cloned in a plasmid vector, and then searched again by base sequencing. The method is as follows.

It is important to adjust the PCR product of gene to a suitable concentration in order to be used as a template in the sequencing reaction. In the invention, 10 ng APC gene was used. To a PCR tube, the PCR product of each exon of APC gene, 3.2 pmol of either one of a forward or reverse primer and 8 µℓ of the reaction mixture (Terminator ready reaction mix; Perkin-Elmer, USA) were added, and sterilized distilled water was added to a 20 µℓ final volume and well mixed. Cycle sequencing reaction was performed for the mixture using a GeneAmp 2700 thermal cycler 25 times of 96°C for 10 seconds, 50°C for 5 seconds and 60 °C for 6 minutes. The obtained reaction product was precipitated with ethanol and centrifuged to remove fluorescence labeled dideoxynucleotide (ddNTPs) in the free primer and the reaction mixture (terminator ready reaction mix), and dried. The thus-obtained DNA was mixed with a mixture of formamide : 25 mM EDTA (pH 8.0) : blue dextran and 10 µℓ of a loading buffer, and denatured in boiling water for 5 minutes. Then, the sample was placed on the ice, and the denatured DNA sample was added to each well of plates, which had been cast previously with 5.5% long ranger gel (BMA, catalogue No., USA). Electrophoresis was performed for 2 to 4 hours and base sequencing was conducted using a software of an ABI Prism 377 automatic sequencer (Perkin-Elmer Biosystems, USA).

The results of the automatic base sequencing for APC gene are shown in Fig. 10.

As results of the sequencing, it was found that normal APC gene was exactly amplified, and therefore the liquid complex of DNA and chitosan according to the method of the invention preserve DNA so stably even after storage at room temperature for a long time to conduct cloning and base sequencing, which means that it is also useful for testing gene mutation and searching cancer.

### Example 8: Investigation of gene cloning and base sequencing possibility for DNA stored in the DNA card for a long time

It was ascertained if for the DNA sample stored as adsorbed on the DNA card at room temperature for a long time, cloning for the product obtained by PCR and automatic base sequencing can be appropriately conducted.

With a template of a genomic DNA sample of a human lung cancer tissue, which was stored as dropped onto the DNA card prepared according to Example 5 for three months, PCR was performed for p53 Tumor Suppressor gene. The product was cloned in a plasmid vector, and then searched again by automatic base sequencing. The method was the same as that of Example 7. The results were shown in Fig. 11.

As the results, it was found that mutagenic p53 gene was exactly amplified (Fig. 11), and that when DNA was stored using the DNA card prepared according to the method of the invention, DNA is preserved to allow cloning and automatic base sequencing therefor even after a long time, so further it is also useful for testing gene mutation and searching cancer.

### Example 9: Southern blotting with a template of a DNA/chitosan complex

In assaying the presence and the amount of a specific gene by a hybridization assay such as Southern blotting and the like using a complex of DNA and chitosan stored according to the method of the invention, the DNA bound to chitosan does not move in agarose gel electrophoresis, so it is difficult to conduct a blotting assay. Therefore, for the blotting assay, the DNA sample should be used with chitosan removed. Therefore, in the invention, a method has been established to remove chitosan without damaging DNA from DNA which is bound to chitosan.

As described above, the water-soluble chitosan is a cationic salt and binds to DNA, so chitosan can be isolated from DNA by treating with a strong cationic salt such as sulfate of SDS (sodium dodecyl sulfate), SOS (sodium octyl sulfate) and CTAB (cetyltrimethyl ammonium bromide). In this case, the cationic salt should be inexpensive and have no effect on the blotting assay without damaging on DNA. Therefore, in the invention, SDS (Sigma Co., USA) was used, which is inexpensive and has no effect on a hybridization reaction between DNA and probe.

A DNA/chitosan complex obtained by mixing a chitosan solution and a pancreatic cancer tissue DNA according to the method of the invention was stored at room temperature for three months. Then, 5 to 10 µg of the DNA/chitosan complex was mixed with 5% (w/v) SDS, and subjected to electrophoresis on agarose gel for Southern blotting. Southern blotting was performed for K-ras gene by a known method (Sambrook J & Russell DW, Molecular cloning: a laboratory manual, 2001). The results were shown in Fig. 12 and Fig. 13, respectively.

From Fig. 12, it was found that in a case of electrophoresis with 5% SDS, the DNA and chitosan were isolated and the DNA moved on the gel.

In addition, Fig. 13 showed results of the Southern blotting that K-ras gene was strongly expressed for both of DNA which was stored at -70°C without binding to chitosan, and DNA stored in the form of chitosan/DNA for 6 months. Therefore, a method was established to perform Southern blotting using DNA stored for a long time according to the DNA storage method of the invention. Furthermore, it was found that DNA stored according to the method of the invention is useful for a hybridization assay.

### Example 10: Investigation of PCR-RFLP assay possibility of gene for DNA stored in the DNA card for a long time

PCR was conducted for a human genomic DNA sample stored using the Type 1 DNA card of the invention, and then it was tested if the genotyping test is possible for it using an RFLP (restriction fragment length polymorphism) assay. The results of genotyping using the RFLP assay after PCR of the Type 1 DNA card were shown in Fig. 8. Furthermore, it was investigated if genotyping is possible using a PCR-RFLP assay for the human sample which was stored as blood in Type 2 DNA card of the present invention. The results were shown in Fig. 9. To prove that the DNA card of the invention can be used practically in gene diagnosis, a gene associated with the attack of cardiovascular diseases was amplified by PCR, and treated with the following specific restriction enzymes. The results were analyzed by electrophoresis. As results, it was found that it has no problem in a search with multiple genotypes.

The present example will be described in detail below.

Using Type 1 DNA card and Type 2 DNA card and the plastic DNA ID card of the invention, the following six genes, which are associated with geriatric diseases, were prepared, respectively in a PCR tube using reaction solutions as shown in the table below. [Table 1]

| composition | eNOS1/2 | MTHFR1/2 | AGT1/2 | ACE1 | ACE2 | AT1R | APOE1/2 |
|---|---|---|---|---|---|---|---|
| D.W. | 20.8 | 20.8 | 19.3 | 19.1 | 17.6 | 20.3 | 17.8 |
| 10 x buffer | 3 | 3 | 3 | 3 | 3 | 3 | 3 |
| 25 mM MgCl₂ | 2 | 2 | 2.5 | 2 | 2 | 3 | 2 |
| 2.5 mM dNTPs | 1 | 1 | 2 | 1.2 | 1.2 | 1.5 | 1 |
| F (10 pmole) | 1 | 1 | 1 | 1 | 1 | 0.5 | 1 |
| R (10 pmole) | 1 | 1 | 1 | 1 | 1 | 0.5 | 1 |
| DMSO | - | - | - | 1.5 | 3 | - | 3 |
| GenePol 5U/l | 0.2 | 0.2 | 0.2 | 0.2 | 0.2 | 0.2 | 0.2 |
| Template DNA | 1 | 1 | 1 | 1 | 1 | 1 | 1 |
| Final volume (µℓ) | 30 | 30 | 30 | 30 | 30 | 30 | 30 |

The PCR tube containing the reaction solution, which was prepared as above, was added to a PE2700 thermal cycler (Perkin Elmer, USA), and was amplified according to each gene as follows.
1. For eNOS1/2, MTHFR1/2, AGT1/2, AT1R, ACE1 gene:
   95°C/5 min, 35 Cycles (95°C/30 sec, 58°C/30 sec, 72°C/40 sec), 72°C /10 min
2. For ACE2 and APOE1/2 gene:
   95°C/5 min, 35 Cycles (95°C/30 sec, 65°C/30 sec, 72°C/40 sec), 72°C /10 min

The thus-obtained PCR product of each gene was confirmed by performing electrophoresis on 1.2% agarose gel containing EtBr. The size of the product of each gene is shown in the following Table 2.

**[Table 2]**

| Gene | Location | Size(bp) |
|---|---|---|
| Apo E | C112R | 330 |
| | R158C | 330 |
| AGT | M235T | 303 |
| | T174M | 354 |
| ACE | D/I | 319/597 |
| | D/D | 335/No amplification |
| ATIB | A1166C | 404 |
| eNOS | G10-T | 676 |
| | E298D | 371 |
| MTHFB | A222V | 198 |
| | A429E | 128 |

To perform RFLP with the thus-obtained PCR product, first, PCR products of other five genes (eNOS, MTHFR, AGT, AT1R and APOE) except only the ACE gene were purified using a DNA Clean & Concentrator kit (Zymo Research Corporation, CA USA) as follows.
1. To the PCR product (about 25 1), the DNA binding solution was added in double volume, i.e., 50 µℓ.
2. To a Zymo spin column, which had been previously provided, all of the mixture according to the above 1 was added and centrifuged at 13,000 rpm for 30 seconds.
3. The solution collected in a collecting tube was removed with a pipet.
4. 200 µℓ of a washing buffer was added to the column and centrifuged at 13,000 rpm for 30 seconds (repeated twice).
5. The remaining washing buffer was completely removed by centrifuging it in an empty tube at 13,000 rpm for 40 seconds.
6. The collecting tube was removed, and a clean 1.5 ml microcentrifuge tube was provided for a new column, and then 20 µℓ of sterilized tertiary distilled water was added thereto, and centrifuged at 13,000 rpm for 40 seconds for elution. Alternatively, sterilized tertiary distilled water heated to 65°C or so may be used.

For the PCR products which are purified and obtained in the above method, a restriction enzyme corresponding to each gene in the table below was prepared under the condition corresponding to each restriction enzyme. Then, they were held for reaction in a water bath at 37°C for 4 to 6 hours, and then, the risk of each gene was searched by electrophoresis on 2.5% agarose gel.

**[Table 3]**

| Gene | Location | Enzyme |
|---|---|---|
| APOE | C112R | AfIIII |
| | R158C | HaeII |
| AGT | M235T | LweI |
| | T174M | Ncol |
| ATIB | A1166C | Ddel |
| ecNOS | G10-T | HincII |
| | E298D | Eco24I |
| MTHFB | A222V | HinfI |
| | A429E | MboII |

### Example 11: Production of PCR kit containing chitosan

As an application of the DNA storage method using chitosan of the invention, a PCR kit was produced wherein Taq polymerase, a primer, dNTP and a buffer and the like were added to one tube with chitosan (all in one tube), and a sample such as DNA or serum was added to the tube to lead to a PCR reaction. Characteristically, this PCR kit can be used for both of DNA storage and PCR assay.

In the present example, using DNA obtained from blood of Type B hepatitis patients, the presence of Type B hepatitis virus was searched with the PCR kit of the invention. The kit can be also applied for cancer diagnosis by diagnosis of cancer-associated gene, or diagnosis of pathogen in Type B virus hepatitis or human papilloma virus infection, sexually transmitted infection, other bacterial infections and the like.

Usage of this kit is as follows.

1 to 3 µg of a DNA template was added to an all-in-one tube containing 10 µℓ of 5 GG one step PCR buffer TM and hot start type Taq polymerase (AmpliTaq Gold, PerkinElmer, USA), 2.0 µℓ of 10 mM dNTP mixture, 1.0 µℓ of each of forward primer and reverse primer (5 pmol) of the target gene, and a 0.1% concentration of chitosan. Distilled water was filled into the kit to a total volume of 50 1, and well mixed, and then PCR was performed. First, reverse transcription reaction was performed at 50°C for 30 minutes, denaturation was conducted at 95°C for 15 minutes, followed by a total of 25 to 40 cycles of 94°C for one minute, 53°C for 40 seconds and 72°C for one minute, then PCR amplification was completed with the reaction of 72°C for 10 minutes. As a result, the presence of DNA of Type B hepatitis virus was found using the PCR kit of the invention (no data attached).

### Example 12: Production of plastic DNA ID card

Fig. 14 is a flow chart which shows a process for preparing a plastic DNA ID card using the DNA card of the invention. Procedures were conducted as in the flow chart of Fig. 14 to develop a DNA ID card of a plastic material.
1. Receiving transported personal sample such as blood or oral cell, DNA card and the like, and personal photograph data (S201).
2. Isolation of genomic DNA from the sample and test of blood typing (S202).
3. Assay of HLA genotype using the extracted genomic DNA, test of STR personal identification gene and analysis for results (S203). Fig. 15 a schematic view which shows a process of identifying personal genotype using 15 combinations of STR test, preparing personal genotype profile, and encoding personal genotype data.
4. Incorporation of a paper DNA card for attachment with the plastic card, and preparation of the frame of the DNA ID card by adding heat and pressure (S205). Fig. 16 is a schematic view which shows incorporation of Type 1 or Type 2 DNA card into a PVC card in the process of preparing the DNA ID card of the invention. As shown in Fig. 16, a PVC soft layer is placed on the lowest end, and Type 1 DNA card or Type 2 DNA card is placed on it. Then, PVC core layer is placed on it, which has a hole as large as the size of the paper DNA card (a hole of the card size must be prepared because of thickness of the paper card and the paper and PVC made of substances different from each other, so they are not incorporated together in incorporation by heating. Specifically, the paper card is inserted onto this hole). On the top of it, a PVC core layer is left which has further two small holes (they are for injecting DNA or blood into the DNA paper card so should be located on the paper card since nearly no empty space is left from incorporation of the card by heating.). Finally, a PVC soft layer is placed on it. Then, heating is conducted under high pressure (100 to 140 bar) at high temperature of 160 to 180°C for 8 to 30 minutes to incorporate them. At this time, the location of the paper DNA card is opposite to that of the magnetic bar, and also opposite to that of photograph transcription. Fig. 17 is a schematic view of one example of the plastic DNA ID card of the invention.
5. Dropping of personal DNA or blood on the plastic DNA ID card and the card for storage, which are produced in S204 (S205). Dropping of the DNA or blood on the plastic DNA ID card is conducted by injecting DNA or blood into the paper DNA card through the hole of the PVC core layer penetrating the PVC soft layer using a needle in. After the injection, the hole caused by the needle is blocked by hologram, sticker and the like.
6. Encoding of the analyzed data on the magnetic bar or the chip of the plastic DNA ID card (S206). The magnetic bar is usually constituted by three tracks. The tract on which English can be written is the first track, which has 78 bits available. The second track has 38 bits available, and the third track has 107 bits available. Therefore, as shown in Fig. 17, brief personal information is encoded on the first track, specific disease code of patients is encoded on the second track (example: C61-Prostatic cancer or N40-Benign prostatic hypertrophy), and results of STR or HLA typing are encoded on the third track. However, in case of connection to bank and the like, the encoding method of the plastic DNA ID card is that gene information is registered only in the main server, and encoded in a way requested by the bank, and data of personal gene information and the like are transmitted from this main server using a POS system. This method is for maximizing further usefulness of the plastic DNA ID card of the invention (S206). Fig. 18 is a schematic view of one example of encoding information on the DNA ID card of the invention.
7. Transcription of personal photograph and brief personal information to carve them on the plastic DNA ID card by the embossing method (S207).
8. Input of personal data into a super computer (for server), assigning of specific personal ID code to the plastic DNA ID card, and setting personal password (S208).
9. Construction and Operation of P0S system to allow a person to access information according to the conditions for security maintenance using an ID and a password, which are recorded in the plastic DNA ID card both at home and abroad if necessary by each person, hospital, each organization or bank and the like (S209).

Fig. 15 is a schematic view of one example of the plastic DNA ID card of the invention. On the front of the card are recorded personal photograph and name, brief personal information (to a level that a hospital can make immediate handle in emergency) and address. On the back of the card, there is a magnetic strip in which personal gene information is encoded, and it is designed to hold signature and DNA storage site.

### Example 13: Experiment of gene amplification from a fragment of the paper DNA card which is stored as incorporated in the plastic DNA ID card

It was ascertained if gene can be amplified from the DNA which was stored in Type 1 or Type 2 paper DNA card, which was incorporated into a plastic DNA ID card, which has been made previously by treatment at high temperature and high pressure, by amplifying eNOS gene, which is one of geriatric disease genes, by PCR. The method was performed in the same manner as in the above Example 10.

Type 1 DNA card and Type 2 DNA card were incorporated, respectively into a plastic DNA ID card which had been prepared at high temperature and high pressure, to have human genomic DNA and blood stored as submerged in each of them. Then, using these plastic DNA ID cards, eNOS gene, which is one of geriatric disease genes, was amplified by PCR for comparison. The results are shown in Fig. 19. Through this experiment, it was found that when Type 2 DNA card for blood absorbing is used in the invention, blood sample must be dropped, and that the plastic cover of the plastic DNA ID card must be removed first in PCR amplification.

Fig. 20 shows results of PCR amplification for eNOS gene, which is one of geriatric disease genes, using a plastic DNA ID card that is treated with high temperature with no pressure artificially in a laboratory, and one that is prepared practically at high temperature and high pressure, to compare the case wherein a sample is added before preparation of the card with the case wherein a sample is added after preparation of the card. Through this experiment, obtained results showed that it is better to drop a sample on previously prepared plastic DNA ID card, regardless that the sample to be stored is DNA or blood.

Type 1 DNA card for DNA dropping and Type 2 DNA card for blood dropping were attached, respectively on a plastic card, which was prepared at high temperature and high pressure to prepare a DNA ID card. To find appropriate concentration and amount of samples to be dropped on each of them, in case of genomic DNA, DNA sample was dropped in 150 ng/? concentration in various volumes using the card fragment preparation as above, and eNOS gene was amplified in PCR and assayed. The results are shown in Fig. 21. Through this experiment, it was found that it is not appropriate to load blood onto Type 1 DNA card (for DNA) and that at least 1.5 µg or so can be dropped when dropping DNA.

The difference was compared and analyzed by amplifying eNDS gene, which is one of geriatric disease genes by PCR, between the case that the card fragment is treated with a washing buffer as a pretreatment process and the case that it is not treated in PCR amplification of the sample stored in a plastic DNA card prepared at high temperature and high pressure. The results are shown in Fig. 22. As a result, PCR amplification was better performed when the card fragment was not treated previously with a washing buffer in PCR using a plastic DNA ID card.

### [Industrial Applicability]

The DNA storing method using the water-soluble chitosan according to the present invention, is excellent in DNA storage efficacy, and makes it possible to store DNA stably at room temperature for a long time on the contrary to the conventional method. Using this method, it is possible to store and carry genomic DNA of big plants and animals, and genomic DNA of fungi, bacteria and virus at room temperature stably for a long time. DNA stored according to the present method is useful for all of gene assays such as PCR and RFLP, cloning, base sequencing, Southern blotting and the like. Furthermore, it can be used broadly in a PCR kit and the like, and it is simple and economical. Especially, the Type 1 DNA card of the invention is very useful for storing and carrying multiple DNA samples at room temperature for a long time, and Type 2 DNA card can store various bio-samples containing DNA such as blood without damage to DNA for a long time at room temperature. In addition, for the sample stored in these DNA cards, various gene assays such as PCR and PCR-RFLP, hybridization, base sequencing, SNP assay and the like can be performed accurately and simply even after a long time, which is very helpful in basic researches and also various clinical treatments. In addition, the DNA ID card of the invention can play a role of DNA bank which stores personal DNAs, and it is also helpful for personal identification and medical treatment such organ transplant and the like by storing the personal gene information as well.

## Claims

1. A method for storing DNA as a form of chitosan/DNA complex prepared by mixing a DNA solution and a water-soluble chitosan solution, wherein the DNA is genomic DNA of animals, plants, fungi, bacteria and virus, and the water-soluble chitosan has the degree of deacetylation of 60% or more, and the molecular weight of 10 kDa to 500 kDa.

2. The method according to claim 1, wherein the concentration of an aqueous solution of water-soluble chitosan is 0.02% (w/v) to 1% (w/v) and the weight ratio of water-soluble chitosan to DNA in the mixture is 1:0.5 or more.

3. The method according to claim 1, wherein the concentration of an aqueous solution of water-soluble chitosan is 0.02% (w/v) to 0.25% (w/v), the concentration of a DNA solution is 1 µg/µl or lower, and the weight ratio of water-soluble chitosan to DNA in the mixture is 1:0.5 to 1:3.

4. The method according to claim 1, wherein the complex of the DNA and chitosan is stored at -70°C to room temperature.

5. The method according to any one of claims 1 to 4, wherein the complex of DNA bound to chitosan is stored as a liquid state.

6. A paper type DNA card for storing DNA, wherein the DNA card is prepared by submerging in a composition comprising water-soluble chitosan and uric acid and drying, and DNA is stored by dropping a DNA solution onto the card as the DNA binds with the water-soluble chitosan, and the DNA is genomic DNA of animals, plants, fungi, bacteria or virus.

7. A paper type DNA card for storing DNA, wherein the DNA card is prepared by submerging in a composition comprising water-soluble chitosan and a cell lysis buffer containing uric acid and drying, and DNA is stored by dropping bio-sample containing genomic DNA onto the card as the DNA binds with the water-soluble chitosan.

8. The paper type DNA card according to claim 6 or 7, wherein the paper is for blotting or chromatography and the thickness thereof is 0.3 to 1.2 mm.

9. The paper type DNA card according to claim 6 or 7, wherein the card is marked with 6, 24, 96 or 384 wells having the size of each well in the diameter of 12.3 cm x 8.1 cm, and DNA or blood sample is dropped and stored on each well.

10. The paper type DNA card according to claim 6, wherein the composition is prepared by mixing 0.1% to 1% (w/v) of water-soluble chitosan with 0.5 mM to 20 mM of uric acid in the volume ratio of 1:1.

11. The paper type DNA card according to claim 7, wherein the cell lysis buffer comprises Tris (8 mM), EDTA (0.5 mM), SDS (0.1% w/v) and uric acid (2 mM).

12. A method for PCR assay on DNA card comprising the steps of:
a) adding a fragment of a DNA card in claim 6 to a PCR tube;
b) adding a TE buffer(10 mM of Tris-Cl, 0.1 mM of EDTA, pH = 8.0) to the tube, and leaving the tube to stand for 5 minutes at room temperature after mixing, and then discarding TE buffer using a pipette;
c) repeating b) step twice;
d) drying the tube for 1 hour at room temperature or for 10 min at 56°C; and
e) adding PCR samples to the tube and amplifying through PCR.

13. A method for PCR assay on DNA card comprising the steps of:
a) adding a fragment of DNA card in claim 7 to a PCR tube;
b) adding a washing buffer (GG purification reagent; 0.5 mM of EDTA, 8 mM of Tris-Cl, 2 mM of uric acid, 1% (w/v) of SDS) to the tube and leaving the tube to stand for 5 minutes at room temperature after mixing, and then discarding washing buffer using a pipette;
c) repeating b) step three times;
d) adding a TE buffer(10 mM of Tris-Cl, 0.1 mM of EDTA, pH = 8.0) to the tube and leaving the tube to stand for 5 min at room temperature after mixing, and then discarding TE buffer using a pipette;
e) repeating d) step twice or three times;
f) drying the tube for one hour at room temperature or for 10 minutes at 56°C; and
g) adding PCR samples to the tube and amplifying through PCR.

14. A method for analysis, wherein the DNA stored according to the storing method of claim 5 or stored on DNA card according to claim 6 or 7 is used in PCR-RFLP, cloning, library synthesis, sequencing analysis or southern blotting.

15. A method for analyzing DNA, wherein the DNA separated from a chitosan/DNA complex as a liquid state stored according to the storing method of claim 5 or the chitosan/DNA complex stored on DNA card according to claim 6 or 7 using a sulfate-based cationic salts is used in gene analysis.

16. The method for analyzing DNA according to claim 15, wherein the sulfate-based cationic salts include SDS (sodium dodecyl sulfate), SOS (sodium octyl sulfate) and CTAB (cetyltrimethyl-ammonium bromide).

17. A PCR kit comprising a tube containing oligo d(T), a primer, Taq DNA polymerase, a reaction buffer solution, dNTP and water-soluble chitosan, wherein the tube additionally contains genomic DNA or serum sample, and the tube is used to perform PCR.

18. A DNA ID card, wherein the mixture of chitosan and genomic DNA of an individual is stored on a portion thereof according to the DNA storing method of claim 1, and the genetic information of the individual is saved on a magnetic bar or an embedded chip.

19. The DNA ID card according to claim 18, wherein the basic material of the DNA ID card is a plastic.

20. A method for preparation of a DNA ID card comprising the steps of:
a) piling DNA paper cards in claim 6 or 7 on a PVC soft layer;
b) piling a first PVC core layer, which has a hole in same size as that of the DNA paper card, on the DNA paper card;
c) piling a second PVC core layer, which has two holes smaller than the hole of the first PVC core layer, over the hole of the first PVC core layer;
d) piling a PVC soft layer on the second PVC core layer; and
e) adding heat and pressure to the piled layers for heat adhesion of each other.

21. The DNA ID card according to claim 19 comprising:
a first PVC soft layer; a DNA paper card; a first PVC core layer having a hole of a size equal to the size of the DNA paper card; a second PVC core layer having holes through which DNA or bio-samples containing DNA are dropped onto the DNA paper card; and a second PVC soft layer,
wherein the magnetic bar is located on the side opposite to the side on which the DNA or bio-sample is stored on the DNA paper card.

22. The DNA ID card according to claim 21, wherein information, disease code, STR or HLA typing of an individual is encoded on the magnetic bar.

23. The DNA ID card according to claim 18, wherein the amount of gDNA dropped on the DNA ID card is 1.5 µg or greater.

24. A method for PCR analysis of DNA ID card, wherein the fragments of DNA ID card of claim 18 is used in the PCR amplification without being treated with a washing buffer.

## Patentansprüche

1. Verfahren zur Lagerung von DNA in Form eines Chitosan/DNA-Komplexes, der durch Mischen einer DNA-Lösung und einer wasserlöslichen Chitosan-Lösung hergestellt wird, wobei die DNA genomische DNA von Tieren, Pflanzen, Pilzen, Bakterien und Viren ist, und das wasserlösliche Chitosan einen Entacetylierungsgrad von 60 % oder mehr und eine Molmasse von 10 kDa bis 500 kDa aufweist.

2. Verfahren gemäß Anspruch 1, wobei die Konzentration einer wässrigen Lösung aus wasserlöslichem Chitosan 0,01 % (Gewicht/Volumen) bis 1 % (Gewicht/Volumen) beträgt und das Gewichtsverhältnis von wasserlöslichem Chitosan zu DNA in der Mischung 1:0,5 oder mehr beträgt.

3. Verfahren gemäß Anspruch 1, wobei die Konzentration der wässrigen Lösung eines wasserlöslichen Chitosans 0,02 % (Gewicht/Volumen) bis 0,25 % (Gewicht pro Volumen) beträgt, die Konzentration der DNA-Lösung 1 µg/µl oder weniger beträgt und das Gewichtsverhältnis des wasserlöslichen Chitosans zu DNA in der Mischung 1:0,5 bis 1:3 beträgt.

4. Verfahren gemäß Anspruch 1, wobei der Chitosan/DNA-Komplex bei Temperaturen von -70 °C bis Raumtemperatur gelagert wird.

5. Verfahren gemäß einem der Ansprüche 1 bis 4, wobei der Chitosan/DNA-Komplex im flüssigen Zustand gelagert wird.

6. DNA-Karte vom Papier-Typ zur Lagerung von DNA, wobei die DNA-Karte durch Eintauchen in eine wasserlösliches Chitosan und Harnsäure enthaltende Zusammensetzung und durch Trocknen herstellbar ist, eine Lagerung der DNA durch Auftropfen einer DNA-Lösung auf die Karte erfolgt, indem sich die DNA an das wasserlösliche Chitosan bindet, und die DNA genomische DNA von Tieren, Pflanzen, Pilzen, Bakterien oder Viren ist.

7. DNA-Karte vom Papier-Typ zur Lagerung von DNA, wobei die DNA-Karte durch Eintauchen in eine Zusammensetzung, die wasserlösliches Chitosan und Harnsäure enthaltende Zelllysepuffer enthält, und Trocknen herstellbar ist, und die DNA gelagert wird, indem eine Bioprobe enthaltend genomische DNA auf die Karte getropft wird, da die DNA sich an das wasserlösliche Chitosan bindet.

8. DNA-Karte vom Papier-Typ gemäß Anspruch 6 oder 7, wobei das Papier für Blotten oder Chromatographie einsetzbar ist und die Dicke davon 0,3 bis 1,2 mm beträgt.

9. DNA-Karte vom Papier-Typ gemäß Anspruch 6 oder 7, wobei die Karte 6, 24, 96 oder 384 Wells aufweist, wobei jedes Well eine Größe mit einem Durchmesser von 12,3 cm x 8,1 cm aufweist und DNA oder Blutproben auf jedes Well getropft und gelagert werden.

10. DNA-Karte vom Papier-Typ gemäß Anspruch 6, wobei die Zusammensetzung durch Mischen von 0,1 % bis 1% (Gewicht/Volumen) eines wasserlöslichen Chitosans mit 0,5 mM bis 20 mM Harnsäure in einem Volumenverhältnis von 1:1 hergestellt ist.

11. DNA-Karte vom Papier-Typ gemäß Anspruch 7, wobei der Zelllysepuffer Tris (8 mM), EDTA (0,5 mM), SDS (0,1 % Gewicht/Volumen) und Harnsäure (2 mM) enthält.

12. Verfahren für ein PCR-Assay auf einer DNA-Karte enthaltend die Schritte:
a) Hinzufügen eines Fragments einer DNA-Karte gemäß Anspruch 6 in ein PCR-Röhrchen;
b) Hinzufügen eines TE-Puffers (10 mM Tris-Cl, 0,1 mM EDTA, pH = 8,0) in das Röhrchen und Stehenlassen des Röhrchens für 5 min bei Raumtemperatur nach Durchmischung; und anschließendem Entfernen des TE-Puffers mittels einer Pipette;
c) zweimaliges Wiederholen von Schritt b);
d) Trocknen des Röhrchens für eine Stunde bei Raumtemperatur oder 10 min bei 56 °C; und
e) Hinzufügen von PCR-Proben in das Röhrchen und Vervielfältigung mittels PCR.

13. Verfahren für ein PCR-Assay auf einer DNA-Karte enthaltend die Schritte:
a) Hinzufügen eines Fragments einer DNA-Karte gemäß Anspruch 7 in ein PCR-Röhrchen;
b) Hinzufügen eines Waschpuffers (GG Aufreinigungsreagenz; 0,5 mM EDTA, 8 mM Tris-Cl, 2 mM Harnsäure, 1 % (Gewicht/Volumen) SDS), in das Röhrchen und Stehenlassen des Röhrchens für 5 min bei Raumtemperatur nach Durchmischung, und anschließendem Entfernen des Waschpuffers mittels einer Pipette;
c) dreimaliges Wiederholen von Schritt b);
d) Hinzufügen eines TE-Puffers (10 mM Tris-Cl, 0,1 mM EDTA, pH = 8,0) in das Röhrchen und Stehenlassen des Röhrchens für 5 min bei Raumtemperatur nach Durchmischung, und anschließendem Entfernen des TE-Puffers mittels einer Pipette;
e) zwei- oder dreimaliges Wiederholen von Schritt d);
f) Trocknen des Röhrchens für eine Stunde bei Raumtemperatur oder für 10 min. bei 56 °C; und
g) Hinzufügen von PCR-Proben in das Röhrchen und Vervielfältigung mittels PCR.

14. Analyseverfahren, wobei DNA, die mittels der Lagerungsmethode gemäß Anspruch 5 oder auf einer DNA-Karte gemäß Ansprüchen 6 oder 7 gelagert ist, für PCR-RFLP, Klonen, Bibliothekserstellung, Sequenzanalyse oder Southern Blots eingesetzt wird.

15. Verfahren zur DNA-Analyse, wobei DNA, die von einem Chitosan/DNA-Komplex im flüssigen Zustand abgetrennt wurde und mittels des Lagerungsverfahrens gemäß Anspruch 5 gelagert wurde oder der Chitosan/DNA-Komplex auf einer DNA-Karte gemäß Anspruch 6 oder 7 gelagert wurde, unter Verwendung eines sulfatbasierten kationischen Salzes in der Analyse von Genen eingesetzt wird.

16. Verfahren zur DNA-Analyse gemäß Anspruch 15, wobei das sulfatbasierte kationische Salz SDS (Natriumdodecylsulfat), SOS (Natriumoctylsulfat) und CTAB (Cetyltrimethylammoniumbromid) einschließt.

17. PCR-Kit enthaltend ein Röhrchen enthaltend Oligo d(T), einen Primer, Taq DNA Polymerase, eine Reaktionspufferlösung, dNTP und wasserlösliches Chitosan, wobei das Röhrchen zusätzlich genomische DNA oder eine Serumprobe enthält, und das Röhrchen für die Durchführung von PCR verwendet wird.

18. DNA-ID-Karte, wobei die Mischung von Chitosan und genomischer DNA einer Einzelperson auf einem Teilbereich davon mittels der DNA-Lagerungsmethode gemäß Anspruch 1 gelagert ist, und die genetische Information der Einzelperson auf einem Magnetstreifen oder einem eingebetteten Chip gesichert ist.

19. DNA-ID-Karte gemäß Anspruch 18, wobei das Grundmaterial der DNA-ID-Karte Kunststoff ist.

20. Verfahren zur Herstellung einer DNA-Kennkarte enthaltend die Schritte:
a) Aufbringen von DNA-Papierkarten gemäß Anspruch 6 oder 7 auf einer weichen PVC-Schicht ;
b) Aufbringen einer ersten PVC-Kernschicht, die ein Loch in der gleichen Größe wie die DNA-Papierkarte aufweist, auf der DNA-Papierkarte;
c) Aufbringen einer zweiten PVC-Kernschicht, die zwei Löcher aufweist, die kleiner als das Loch der ersten PVC-Kernschicht sind, über das Loch der ersten PVC-Kernschicht;
d) Aufbringen einer weichen PVC-Schicht auf die zweite PVC-Kernschicht; und
e) Beaufschlagung der aufgebrachten Schichten mit Wärme und Druck, so dass sie durch Hitzeadhäsion miteinander verbunden sind.

21. DNA-ID-Karte gemäß Anspruch 19, enthaltend:
eine erste weiche PVC-Schicht; eine DNA-Papierkarte; eine erste PVC-Kernschicht mit einem Loch, das eine Größe aufweist, die der der DNA-Papierkarte entspricht; eine zweite PVC-Kernschicht mit Löchern, durch die DNA oder Bioproben, die DNA enthalten, auf die DNA-Papierkarte aufgetropft werden; eine zweite weiche PVC-Schicht, wobei der Magnetstreifen auf der Seite gegenüber der Seite, auf der die DNA oder Bioprobe gelagert ist, auf der DNA-Papierkarte angeordnet ist.

22. DNA-ID-Karte gemäß Anspruch 21, wobei Informationen, Krankheitcodes, STR- oder HLA-Typisierung einer Einzelperson auf dem Magnetstreifen verschlüsselt ist.

23. DNA-ID-Karte gemäß Anspruch 18, wobei die Menge der gDNA, die auf die DNA-ID-Karte getropft wurde, 1,5 µg oder größer ist.

24. Ein Verfahren zur PCR-Analyse von DNA-ID-Karte, wobei die Fragmente der DNA-ID-Karte gemäß Anspruch 18 für die PCR-Vervielfältigung verwendet werden, ohne dass sie mit einem Waschpuffer behandelt wurden.

## Revendications

1. Procédé servant à stocker de l'ADN sous la forme d'un complexe de chitosane/ADN préparé en mélangeant une solution d'ADN et une solution de chitosane hydrosoluble, dans lequel l'ADN est un ADN génomique d'animaux, de plantes, de champignons, de bactéries ou de virus, et le chitosane hydrosoluble a un degré de désacétylation de 60 % ou plus et un poids moléculaire de 10 kDa à 500 kDa.

2. Procédé selon la revendication 1, dans lequel la concentration d'une solution aqueuse de chitosane hydrosoluble est de 0,02 % (p/v) à 1 % (p/v) et le rapport pondéral du chitosane hydrosoluble à l'ADN dans le mélange est de 1:0,5 ou plus.

3. Procédé selon la revendication 1, dans lequel la concentration d'une solution aqueuse de chitosane hydrosoluble est de 0,02 % (p/v) à 0,25 % (p/v), la concentration d'une solution d'ADN est de 1 µg/µl ou moins, et le rapport pondéral du chitosane hydrosoluble à l'ADN dans le mélange est de 1:0,5 à 1:3.

4. Procédé selon la revendication 1, dans lequel le complexe de l'ADN et du chitosane est stocké à une température entre -70 °C et la température ambiante.

5. Procédé selon l'une quelconque des revendications 1 à 4, dans lequel le complexe d'ADN lié au chitosane est stocké à l'état liquide.

6. Carte d'ADN de type papier servant à stocker l'ADN, dans laquelle la carte d'ADN est préparée par immersion dans une composition comprenant du chitosane hydrosoluble et de l'acide urique et par séchage, et l'ADN est stocké par dégouttage d'une solution d'ADN sur la carte comme l'ADN se lie au chitosane hydrosoluble, et l'ADN est de l'ADN génomique d'animaux, de plantes, de champignons, de bactéries ou de virus.

7. Carte d'ADN de type papier servant à stocker l'ADN, dans laquelle la carte d'ADN est préparée par immersion dans une composition comprenant du chitosane hydrosoluble et un tampon de lyse cellulaire contenant de l'acide urique et par séchage et l'ADN est stocké par dégouttage du bio-échantillon contenant de l'ADN génomique sur la carte comme l'ADN se lie au chitosane hydrosoluble.

8. Carte d'ADN de type papier selon la revendication 6 ou 7, dans laquelle le papier sert au buvardage ou à la chromatographie et son épaisseur est de 0,3 à 1,2 mm.

9. Carte d'ADN de type papier selon la revendication 6 ou 7, dans laquelle la carte est marquée de 6, 24, 96 ou 384 puits dont la taille individuelle est un diamètre de 12,3 cm x 8,1 cm, et l'ADN ou l'échantillon sanguin est dégoutté et stocké dans chaque puits.

10. Carte d'ADN de type papier selon la revendication 6, dans laquelle la composition est préparée en mélangeant 0,1 % à 1 % (p/v) de chitosane hydrosoluble entre 0,5 mM et 20 mM d'acide urique dans les proportions volumiques de 1:1.

11. Carte d'ADN de type papier selon la revendication 7, dans laquelle le tampon de lyse cellulaire comprend du Tris (8 mM), de l'EDTA (0,5 mM), du SDS (0,1 % p/v) et de l'acide urique (2 mM).

12. Procédé pour essai PCR sur carte d'ADN comprenant les étapes consistant à :
a) ajouter un fragment d'une carte d'ADN selon la revendication 6 à un tube à PCR ;
b) ajouter un tampon de TE (10 mM de Tris-Cl, 0,1 mM d'EDTA, pH = 8,0) dans le tube et laisser reposer le tube à température ambiante pendant 5 minutes après mélange, puis jeter le tampon de TE à l'aide d'une pipette ;
c) répéter l'étape b) deux fois ;
d) sécher le tube pendant 1 heure à température ambiante ou pendant 10 mn à 56 °C ; et
e) ajouter des échantillons de PCR dans le tube et amplifier par PCR.

13. Procédé pour essai PCR sur carte d'ADN comprenant les étapes consistant à :
a) ajouter un fragment d'une carte d'ADN selon la revendication 7 à un tube à PCR ;
b) ajouter un tampon de lavage (réactif de purification GG ; 0,5 mM d'EDTA, 8 mM de Tris-Cl, 2 mM d'acide urique, 1 % (p/v) de SDS) dans le tube et laisser reposer le tube à température ambiante pendant 5 minutes après mélange, puis jeter le tampon de lavage à l'aide d'une pipette ;
c) répéter l'étape b) trois fois ;
d) ajouter un tampon de TE (10 mM de Tris-Cl, 0,1 mM d'EDTA, pH = 8,0) dans le tube et laisser reposer le tube à température ambiante pendant 5 minutes après mélange, puis supprimer le tampon de TE en utilisant une pipette ;
e) répéter l'étape d) deux ou trois fois ;
f) sécher le tube pendant une heure à température ambiante ou pendant 10 mn à 56 °C; et
g) ajouter des échantillons de PCR dans le tube et amplifier par PCR.

14. Procédé d'analyse, dans lequel l'ADN stocké selon le procédé de stockage de la revendication 5 ou stocké sur une carte d'ADN selon la revendication 6 ou 7 est utilisé en PCR-RFLP, en clonage, en synthèse de banque, en analyse de séquençage ou en buvardage de Southem.

15. Procédé d'analyse d'ADN, dans lequel l'ADN séparé d'un complexe de chitosane/ADN à l'état liquide stocké selon le procédé de stockage de la revendication 5 ou le complexe de chitosane/ADN stocké sur la carte d'ADN selon la revendication 6 ou 7 utilisant des sels cationiques à base de sulfate est utilisé en analyse génique.

16. Procédé d'analyser l'ADN selon la revendication 15, dans lequel les sels cationiques à base de sulfate comprennent le SDS (dodécylsulfate de sodium), le SOS (octylsulfate de sodium) et le CTAB (bromure de cétyltriméthylammonium).

17. Nécessaire à PCR comprenant un tube contenant un oligo d(T), une amorce, l'ADN polymérase Taq, une solution de tampon réactionnel, un dNTP et un chitosane hydrosoluble, dans laquelle le tube contient de plus un ADN génomique ou un échantillon de sérum, et on utilise le tube pour effectuer la PCR.

18. Carte d'identité d'ADN, dans laquelle le mélange de chitosane et d'ADN génomique d'un individu est stocké sur une partie de celle-ci selon le procédé de stockage d'ADN de la revendication 1, et les informations génétiques de l'individu sont stockées sur une barre magnétique ou sur une puce incorporée.

19. Carte d'identité d'ADN selon la revendication 18, dans laquelle le matériau de base de la carte d'identité d'ADN est un plastique.

20. Procédé pour la préparation d'une carte d'identité d'ADN comprenant les étapes consistant à :
a) empiler des cartes d'ADN en papier selon la revendication 6 ou 7 sur une couche de PVC souple ;
b) empiler une première couche centrale de PVC qui a un trou de la même taille que celui de la carte d'ADN en papier sur la carte d'ADN en papier ;
c) empiler une seconde couche centrale de PVC qui a deux trous plus petits que le trou de la première couche centrale de PVC, par-dessus le trou de la première couche centrale de PVC ;
d) empiler une couche de PVC souple sur la seconde couche centrale de PVC;
et
e) ajouter de la chaleur et de la pression aux couches empilées pour une adhérence thermique entre elles.

21. Carte d'identité d'ADN selon la revendication 19 comprenant :
une première couche de PVC souple ; une carte d'ADN en papier ; une première couche centrale de PVC ayant un trou d'une taille égale à la taille de la carte d'ADN en papier ; une seconde couche centrale de type PVC ayant des trous à travers lesquels l'ADN ou les bio-échantillons contenant de l'ADN sont dégouttés sur la carte d'ADN en papier et une seconde couche de PVC souple,
dans laquelle la barre magnétique se trouve sur le côté opposé au côté sur lequel l'ADN ou le bio-échantillon est stocké sur la carte d'ADN de type papier.

22. Carte d'identité d'ADN selon la revendication 21, dans laquelle on code des informations, un code de maladie, un typage STR ou HLA d'un individu sur la barre magnétique.

23. Carte d'identité d'ADN selon la revendication 18, dans laquelle la quantité d'ADNg dégouttée sur la carte d'identité d'ADN est de 1,5 µg ou plus.

24. Procédé d'analyse par PCR d'une carte d'identité d'ADN, dans lequel on utilise les fragments de carte d'identité d'ADN selon la revendication 18 dans l'amplification par PCR sans les traiter avec un tampon de lavage.
